(19) Europäisches Patentamt
European Patent Office
Office européen des brevets

(11) EP 3 143 059 B1

(12) **EUROPEAN PATENT SPECIFICATION**

(45) Date of publication and mention of the grant of the patent:
**10.01.2018 Bulletin 2018/02**

(21) Application number: **15722991.5**

(22) Date of filing: **07.05.2015**

(51) Int Cl.:
***C08G 18/00*** (2006.01) ***C08G 18/76*** (2006.01)
***C08G 18/71*** (2006.01) ***C08G 59/40*** (2006.01)
***C08G 18/16*** (2006.01) ***C07D 263/22*** (2006.01)

(86) International application number:
**PCT/EP2015/060060**

(87) International publication number:
**WO 2015/173110 (19.11.2015 Gazette 2015/46)**

(54) **CATALYSTS FOR THE SYNTHESIS OF OXAZOLIDINONE COMPOUNDS**
KATALYSATOREN FÜR DIE SYNTHESE VON OXAZOLIDINONVERBINDUNGEN
CATALYSEURS POUR LA SYNTHÈSE DE COMPOSÉS D'OXAZOLIDINONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2014 EP 14167821**
**13.02.2015 EP 15154979**

(43) Date of publication of application:
**22.03.2017 Bulletin 2017/12**

(73) Proprietor: **Covestro Deutschland AG**
**51373 Leverkusen (DE)**

(72) Inventors:
- **MÜLLER, Thomas Ernst**
**52074 Aachen (DE)**
- **GÜRTLER, Christoph**
**50735 Köln (DE)**
- **BASU, Susmit**
**52074 Aachen (DE)**
- **RANGHEARD, Claudine**
**NL-6211 CB Maastricht (NL)**
- **WEBER, Anna**
**50939 Köln (DE)**
- **VOGT, Henning**
**52066 Aachen (DE)**
- **LEITNER, Walter**
**52074 Aachen (DE)**

(74) Representative: **Levpat**
**c/o Covestro AG**
**Gebäude 4825**
**51365 Leverkusen (DE)**

(56) References cited:
**US-A- 5 480 958**
**US-A1- 2010 227 090**
**US-A1- 2014 121 299**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 143 059 B1

## Description

The present invention relates to a method for the production of oxazolidinone compounds with low colour intensity, comprising the step of reacting an isocyanate compound with an epoxide compound in the presence of a catalyst which is free of halide anions. The invention further relates to a method for the production of oligooxazolidinone and/or polyoxazolidinone compounds, comprising the step of reacting a polyisocyanate compound with a polyepoxide compound in the presence of said catalyst. The invention further relates to oligooxazolidinone and/or polyoxazolidinone compounds with low colour intensity, obtainable by a method according to the invention.

Oxazolidinones are widely used structural motifs in pharmaceutical applications, and the cycloaddition of epoxides and isocyanates seems to be a convenient one-pot synthetic route to produce them. Expensive catalysts, reactive polar solvents, long reaction times and low chemoselectivities are common in early reports for the synthesis of oxazolidinones (M. E. Dyen and D. Swern, Chem. Rev., 67, 197 (1967); X. Zhang and W. Chen, Chem. Lett., 39, 527 (2010); M.T. Barros and A.M.F. Phillips, Tetrahedron: Asymmetry, 21, 2746 (2010); H.-Y. Wu, J.-C. Ding and Y.-K. Liu, J. Indian Chem. Soc., 80, 36 (2003); C. Qian and D. Zhu, Synlett, 129 (1994)). Therefore, the development of new catalyst systems is desirable to achieve high chemoselectivities.

US 5 480 958 A describes the synthesis of polyepoxide resins incorporating epoxy terminated urethanes for the use as toughener. Herein the used epoxy terminated urethane prepolymer contains oxazolidinone structures. Said prepolymers are built up from polyisocyanates, carrying isocyanate end groups, and diepoxides and synthesised solvent free at a temperature of 160 °C catalysed by anhydrous potassium acetate. No other potential catalysts are given. The selectivity towards oxazolidinone formation is not described.

US 2014/121299 A1 describes the synthesis of oxazolidinone containing thermosettable epoxysystems as tougheners. The method for the production of oxazolidinone is based on the combination of an isocyanate compound and a polyether glycol epoxy resin. The reaction is performed at 135°C to 180 °C with a catalyst. Possible catalysts for the reaction to oxazolidinone are uncharged strong, non-nucleophilic amine based bases such as DBU. The oxazolidinone is formed in selectivities of about 95% percent (compared to isocyanurate).

US 2010/227090 A1 discloses a method for producing an epoxy terminated polyoxazolidinone from a hydroxyl containing epoxide and an isocyanate can be cured to form high temperature stable thermosets. The reaction is performed at 160°C with a semi-batch addition. The catalyst used in the examples is 2-phenyl imidazole. Other possible catalysts named are tertiary amines and phosphines, as well as ammonium and phosphonium salts. The only halogenide free salts disclosed are the corresponding acetates. There is no mentioning of thermoplastic behaviour.

Unpublished European Patent Application No. 12192611.7 relates to a method for the production of oxazolidinone compounds, comprising the step of reacting an isocyanate compound with an epoxide compound in the presence of a Lewis acid catalyst. All catalysts used in the examples ($SmI_3$, LiBr, $Ph_4SbBr$ $Ph_4PBr$) contain a halide anion.

US 3687897 discloses a process for the preparation of oxazolidinone compounds by reacting an isocyanate with an epoxide in presence of a phosphonium catalyst such as tetrabutylphosphonium bromide. The examples disclose the use of a tetrabutylphosphonium bromide or iodide catalyst. The chemoselectivity as well as the use of catalysts free of halide anions have not been disclosed.

In all disclosures, the catalyst comprises a halide anion. The use of halide salts, in particular bromide and iodide salts, has the disadvantage that the resulting products tend to have a high colour intensity and may get further discoloured upon exposure to light. During the production of the oxazolidinone and/or polyoxazolidinone compounds, the presence of halides may cause corrosion and necessitates the use of special corrosion-resistant equipment. For the same reason, special corrosion-resistant equipment is required for processing of the resulting oxazolidinone and/or polyoxazolidinone compounds or the catalyst needs to be removed from the oxazolidinone and/or polyoxazolidinone products beforehand.

The object was therefore to identify catalysts for the production of oxazolidinone and/or polyoxazolidinone compounds by reacting isocyanate compounds with epoxide compounds, which lead to products having low colour intensity. It would further be desirable if such catalysts provide a high chemoselectivity to the oxazolidinone product. In the manufacture of polyoxazolidinones by reacting diisocyanates with diepoxides in the presence of the catalyst according to the invention, certain side reactions should be avoided to obtain polymers with useful properties. Such undesired side reactions include for example the trimerisation of isocyanate to isocyanurate groups, the formation of carbodiimides and homo-polymers of the epoxides. The specifications concerning a high chemoselectivity to the oxazolidinone compound are particularly stringent, if polyoxazolidinone compounds with an almost linear polymer backbone and thermoplastic properties are desired. In particular, the formation of isocyanurate groups should be supressed as this side reaction may result in crosslinking of the polymer chains.

Surprisingly, this was achieved by using catalysts free of halide anions, which comprise a conjugated trigonal-planar anionic moiety having a central atom selected from the elements C or N and three further substituent atoms, independently selected from the elements C, N, O, S and/or P, and wherein the overall charge state of the anionic moiety is -1 or -2, in the reaction of isocyanate compounds with epoxide compounds.

Therefore the subject matter of the present invention is a method for the production of oxazolidinone compounds,

comprising the step of reacting an epoxide compound and an isocyanate compound in the presence of a catalyst, characterized in that the catalyst

is free of halide anions and

comprises a conjugated trigonal-planar anionic moiety having a central atom selected from the elements C or N and three further substituent atoms, independently of one another selected from the elements C, N, O, S and/or P, and wherein the overall charge state of the conjugated trigonal-planar anionic moiety is -1 or -2.

The resulting products have a significantly reduced intensity in colour compared to oxazolidinone compounds obtained by the use of a catalyst known from the state of the art, in particular tetraphenylphosphonium bromide and tetraphenylphosphonium chloride. Furthermore, the catalysts according to the invention enable the production of the oxazolidinone compounds with high chemoselectivity.

As used herein, the term "oxazolidinone compound" is meant to denote oxazolidinone compounds obtainable by the reaction of an isocyanate compound with an epoxide compound. The term "oxazolidinone compound" is meant to include polyoxazolidinone compounds with at least two oxazolidinone moieties in the molecule obtainable by the reaction of a polyisocyanate with a polyepoxide.

As used herein, the term "isocyanate compound" is meant to denote monoisocyanate compounds, polyisocyanate compounds (having two or more NCO groups), NCO-terminated biuretsisocyanurates, uretdiones, carbamates and NCO-terminated prepolymers. The term "monoisocyanate compound" is meant to denote isocyanate compounds having one isocyanate group. The term "polyisocyanate compound" is meant to denote isocyanate compounds having at least two isocyanate groups. The term "diisocyanate compound" is meant to denote polyisocyanate compounds having two isocyanate groups.

As used herein, the term "epoxide compound" is meant to denote monoepoxide compounds, polyepoxide compounds (having two or more epoxide groups) and epoxide-terminated prepolymers. The term "monoepoxide compound" is meant to denote epoxide compounds having one epoxy group. The term "polyepoxide compound" is meant to denote epoxide compounds having at least two epoxy groups. The term "diepoxide compound" is meant to denote epoxide compounds having two epoxy groups.

Examples of suitable monoisocyanate compounds are methyl isocyanate, ethyl isocyanate, n-propyl isocyanate, isopropyl isocyanate, n-butyl isocyanate, isobutyl isocyanate, tert-butyl isocyanate, n-pentyl isocyanate, n-hexyl isocyanate, cyclohexyl isocyanate, ω-chlorohexamethylene isocyanate, n-heptyl isocyanate, n-octyl isocyanate, iso-octyl isocyanate, 2-ethyl hexyl isocyanate, 2-norbornyl methyl isocyanate, nonyl isocyanate, 2,3,4-trimethylcyclohexyl isocyanate, 3,3,5-trimethylcyclohexyl isocyanate, decyl isocyanate, undecyl isocyanate, dodecyl isocyanate, tridecyl isocyanate, tetradecyl isocyanate, pentadecyl isocyanate, hexadecyl isocyanate, octadecyl isocyanate, stearyl isocyanate, 3-butoxypropyl isocyanate, 3-(2-ethylhexyloxy)-propyl isocyanate, 6-chlorohexyl isocyanate benzyl isocyanate, phenyl isocyanate, *ortho-, meta-, para*-tolyl isocyanate, dimethylphenyl isocyanate (technical mixture and individual isomers), 4-pentylphenyl isocyanate, 4-cyclohexylphenyl isocyanate, 4-dodecylphenyl isocyanate, *ortho-, meta-, para*-methoxyphenyl isocyanate, chlorophenyl isocyanate (2,3,4-isomers), the different dichlorophenyl isocyanate isomers, 4-nitrophenyl isocyanate, 3-trifluoromethylphenyl isocyanate, 1-naphthyl isocyanate.

Preferred monoisocyanate compounds are benzyl isocyanate, phenyl isocyanate, *ortho-, meta-, para*-tolyl isocyanate, dimethylphenyl isocyanate (technical mixture and individual isomers), 4-cyclohexylphenyl isocyanate and *ortho-, meta-, para*-methoxyphenyl isocyanate.

A mixture of two or more of the aforementioned monoisocyanate compounds can also be used.

Examples of polyisocyanate compounds are tetramethylene diisocyanate, hexamethylene diisocyanate (HDI), 2-methylpentamethylene diisocyanate, 2,2,4-trimethylhexamethylene diisocyanate (THDI), dodecamethylene diisocyanate, 1,4-diisocyanatocyclohexane, 3-isocyanatomethyl-3,3,5-trimethylcyclohexyl isocyanate (isophorone diisocyanate, IPDI), 4,4'-diisocyanatodicyclohexylmethane ($H_{12}$-MDI), 4,4'-diisocyanato-3,3'-dimethyldicyclohexylmethane, 4,4'-diisocyanato-2,2-dicyclohexylpropane, poly(hexamethylene diisocyanate), octamethylene diisocyanate, tolylene-α,4-diisocyanate, poly(propylene glycol) tolylene-2,4-diisocyanate terminated, poly(ethylene adipate) tolylene-2,4-diisocyanate terminated, 2,4,6-trimethyl-1,3-phenylene diisocyanate, 4-chloro-6-methyl-1,3-phenylene diisocyanate, poly[1,4-phenylene diisocyanate-co-poly(1,4-butanediol)] diisocyanate, poly(tetrafluoroethylene oxide-co-difluoromethylene oxide) α,ω-diisocyanate, 1,4-diisocyanatobutane, 1,8-diisocyanatooctane, 1,3-bis(1-isocyanato-1-methylethyl)benzene, 3,3'-dimethyl-4,4'-biphenylene diisocyanate, naphthalene-1,5-diisocyanate, 1,3-phenylene diisocyanate, 1,4-diisocyanatobenzene, 2,4- or 2,5- or 2,6-diisocyanatotoluene (TDI) or mixtures of these isomers, 4,4'-, 2,4'- or 2,2'-diisocyanatodiphenylmethane (MDI) or mixtures of these isomers, 4,4'-, 2,4'- or 2,2'-diisocyanato-2,2-diphenylpropane-p-xylene diisocyanate and α,α,α',α'-tetramethyl- m- or -p-xylene diisocyanate (TMXDI), mixtures thereof or biurets, isocyanurates, carbamates or uretdiones of the aforementioned isocyanates.

Preferred polyisocyanates are hexamethylene diisocyanate (HDI), 3-isocyanatomethyl-3,3,5-trimethylcyclohexyl isocyanate (isophorone diisocyanate, IPDI), 4,4'-diisocyanatodicyclohexylmethane ($H_{12}$-MDI), 2,4- or 2,5- or 2,6-diisocyanatotoluene (TDI) or mixtures of these isomers, 4,4'-, 2,4'- or 2,2'-diisocyanatodiphenylmethane (MDI) or mixtures of these isomers.

A mixture of two or more of the aforementioned polyisocyanates can also be used.

Examples of monoepoxide compounds are ethylene oxide, propylene oxide, 1,2-butene oxide, 2,3-butene oxide, butadiene monoepoxide, 1,2-hexene oxide, cyclohexene oxide, vinylcyclohexene monoxide, limonene monoxide, oxides of C10 - C18 alpha-olefins, styrene oxide, the epoxides of unsaturated fatty acid C1 - C18 alkyl esters, methyl glycidyl ether, ethyl glycidyl ether, propyl glycidyl ether, butyl glycidyl ether, pentyl glycidyl ether, hexyl glycidyl ether, cyclohexyl glycidyl ether, octyl glycidyl ether, 2-ethylhexyl glycidyl ether, C10 - C18 alkyl glycidyl ether, allyl glycidyl ether, benzyl glycidyl ether, phenyl glycidyl ether, 4-tert-butylphenyl glycidyl ether, 1-naphthyl glycidyl ether, 2-naphthyl glycidyl ether, 2-chlorophenyl glycidyl ether, 4-chlorophenyl glycidyl ether, 4-bromophenyl glycidyl ether, 2,4,6-trichlorophenyl glycidyl ether, 2,4,6-tribromophenyl glycidyl ether, pentafluorophenyl glycidyl ether, o-cresyl glycidyl ether, m-cresyl glycidyl ether, p-cresyl glycidyl ether, glycidyl acetate, glycidyl cyclohexylcarboxylate, glycidyl benzoate, and *N*-glycidyl phthalimide.

Preferred monoepoxide compounds are ethylene oxide, propylene oxide, 1,2-butene oxide, 2,3-butene oxide, styrene oxide, butyl glycidyl ether, benzyl glycidyl ether, phenyl glycidyl ether, p-tolyl glycidyl ether, 4-tert-butylphenyl glycidyl ether.

A mixture of the aforementioned monoepoxide compounds can also be used.

Diepoxide compounds are for example butadiene diepoxide, vinylcyclohexene diepoxide, limonene diepoxide, the diepoxides of double unsaturated fatty acid C1 - C18 alkyl esters, ethylene glycol diglycidyl ether, di(ethylene glycol) diglycidyl ether, poly(ethylene glycol) diglycidyl ether, propylene glycol diglycidyl ether, di(propylene glycol) diglycidyl ether, poly(propylene glycol) diglycidyl ether, neopentyl glycol diglycidyl ether, polybutadiene diglycidyl ether, 1,6-hexanediol diglycidyl ether, hydrogenated bisphenol-A diglycidyl ether, 1,2-dihydroxybenzene diglycidyl ether, resorcinol diglycidyl ether, 1,4-dihydroxybenzene diglycidyl ether, bisphenol-A diglycidyl ether, diglycidyl ethers of polybutadiene - bisphenol-A - block-copolymers, diglycidyl o-phthalate, diglycidyl isophthalate, diglycidyl terephthalate.

Preferred diepoxide compounds are butadiene diepoxide, the diepoxides of double unsaturated fatty acid C1 - C18 alkyl esters, ethylene glycol diglycidyl ether, di(ethylene glycol) diglycidyl ether, poly(ethylene glycol) diglycidyl ether, propylene glycol diglycidyl ether, di(propylene glycol) diglycidyl ether, poly(propylene glycol) diglycidyl ether, neopentyl glycol diglycidyl ether, 1,6-hexanediol diglycidyl ether, hydrogenated bisphenol-A diglycidyl ether, 1,2-dihydroxybenzene diglycidyl ether, resorcinol diglycidyl ether, 1,4-dihydroxybenzene diglycidyl ether, bisphenol-A diglycidyl ether, diglycidyl o-phthalate, diglycidyl isophthalate, diglycidyl terephthalate.

A mixture of two or more the aforementioned diepoxides can also be used.

Polyepoxide compounds are for example glycerol polyglycidyl ether, trimethylolpropane polyglycidyl ether, pentaerythritol polyglycidyl ether, diglycerol polyglycidyl ether, polyglycerol polyglycidyl ether, sorbitol polyglycidyl ether.

A mixture of one or more polyepoxide compounds and/or one or more of the aforementioned diepoxide compounds can also be used.

The catalyst according to the invention is a compound free of halide anions and comprising a conjugated trigonal-planar anionic moiety having a central atom selected from the elements C or N and three further substituent atoms, selected independently of one another from the elements C, N, O, S and/or P, wherein the overall charge state of the anionic moiety is -1 or -2. The term "trigonal-planar" in the context of this invention is used in accordance with the valence-shell electron pair repulsion model (VSEPR model) as described for example in Holleman, A. F.; Wiberg, E.; "Inorganic Chemistry"; Academic Press: San Diego, 2001. In this context, the term "trigonal-planar" describes a three dimensional geometrical arrangement, in which the three substituent atoms are arranged around the central atom in a triangular manner, and the central atom is located within the triangular plane. It is to be understood that within the scope of the invention, small deviations from the planar arrangement are accepted. Thus, the central atom may be slightly dislocated from the plane so that any bond between a substituent atom and the central atom forms an angle of no more than 30° with the triangular plane. The term "conjugated" as used herein is meant to denominate an arrangement, in which all four atoms are part of a common, conjugated $\pi$-system. The condition "conjugated" is fulfilled if a mesomeric structure can be formulated in which one of the three substituent atoms is bound to the central atom by a double bond, and the remaining substituent atoms carry either a lone electron pair, or are bound to further substituents by a double bond, or are part of a further $\pi$-system, such as an aromatic system.

The anionic moiety contained in the catalyst according to the invention has an overall charge state of -1 or -2. The anionic moiety can be a mono- or double-charged anion without any covalent bonds to further binding partners, or be part of a larger molecular structure, to which it is connected by covalent bonds via one or two of the substituent atoms contained in the anionic moiety. One or two of the substituent atoms can also be connected to a larger molecular structure in such a way that they form, together with the central atom of the anionic moiety, a cyclic structure within the larger molecular framework.

The following examples are given to further elucidate the meaning of the term "conjugated trigonal-planar anionic moiety" in the context of the invention, it is however to be understood that the invention is not limited to these examples. Formulae (I) to (III) are examples for "conjugated trigonal-planar anionic moiety" structures whereas formula (IV) represents a "non-conjugated trigonal-planar anionic moiety".

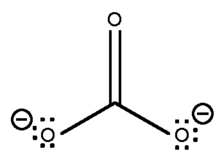

(I)

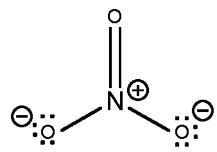

(II)

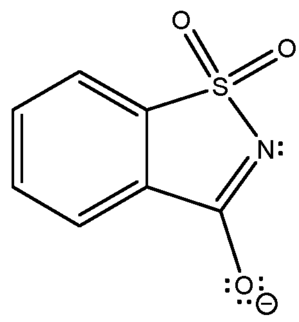

(III)

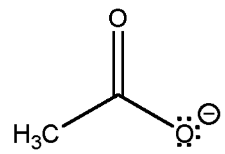

(IV)

The structure shown in formula I (carbonate anion) features a double bond between one oxygen atom (substituent atom) and the central carbon atom, which is bound to two further oxygen atoms (substituent atoms), each of which carries a lone electron pair. The overall charge is -2. The carbonate is thus a conjugated trigonal-planar anionic moiety.

The structure shown in formula II (nitrate anion) features a double bond between one oxygen atom (substituent atom) and the central nitrogen atom, which is bound to two further oxygen atoms (substituent atoms), each of which carries a lone electron pair. The overall charge is -1. The nitrate is thus a conjugated trigonal-planar anionic moiety.

The structure shown in formula III (saccharinate anion) features a double bond between a nitrogen atom (substituent atom) and a central carbon atom, which is bound on one side to an oxygen atom (substituent atom) carrying a lone pair, and on the other side to a carbon atom (substituent atom) which is part of an aromatic $\pi$-system. The overall charge is -1. The N-C(O)-C-moiety is a conjugated trigonal-planar anionic moiety and has an overall charge of -1. The anionic moiety is part of a larger molecular framework. The saccharinate anion thus comprises a conjugated trigonal-planar anionic moiety.

The structure shown in formula IV (acetate anion) features a double bond between one oxygen atom (substituent atom) and the central carbon atom, which is bound to another oxygen atom (substituent atom) carrying a lone electron pair. The third substituent atom (carbon atom) does neither carry a lone electron pair, nor is it bound with a double bond to further binding partners or part of another $\pi$-system, such as an aromatic system. The acetate anion is therefore not a conjugated trigonal-planar anionic moiety.

The catalyst according to the invention can also contain more than one anionic moiety within one molecule. It is preferred that all anionic moieties contained in the catalyst according to the invention have a conjugated trigonal-planar structure as specified above.

The catalyst according to the invention is overall neutral in charge. It thus contains an appropriate number of positive charges to compensate for the negative charge or charges of the anionic moiety or moieties. The positive charges can be present in the form of one or more suitable separate counter cations, which are not bound via one or more covalent bonds to the anionic moiety. In this case, the catalyst according to the invention is a salt comprising separate cations and anions, whereby the anions comprise at least one conjugated trigonal-planar anionic moiety as specified above. The positive charges can also be present as cationic moieties in the same molecule as the anionic moieties, however, the charges are then strictly separated, i.e. the positive and the negative charge are not in conjugation to each other. In this case, the catalyst of the invention is a zwitterion.

In one preferred embodiment of the invention the catalyst is a salt comprising separate cations and anions.

In one embodiment of the invention the anionic moiety in the catalyst has the general formula (V),

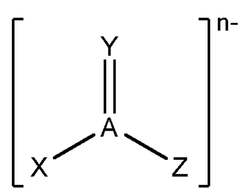

(V)

wherein

n is an integer of 1 or 2,
A is C or N+,

X is $O^-$, OH, $S^-$, SH, OR, SR, NRR', NHR, $N(R)^-$, $N(SO_2R)^-$ or PRR',
wherein R, R' independently of one another are linear or branched, saturated, mono- or polyunsaturated aliphatic, cycloaliphatic or aromatic groups comprising 1 to 20 carbon atoms, optionally substituted with one or more heteroatom containing groups, wherein the heteroatom is selected from N, O, S, P, Si, F and/or Cl,
wherein R, R' can be connected within a (hetero)aliphatic or (hetero)aromatic mono- or polycyclic ring comprising 1 to 20 carbon atoms and/or heteroatoms selected from the group N, P, O and/or S optionally substituted with one or more heteroatom containing groups, wherein the heteroatom is selected from N, O, S, P, Si, F and/or Cl, or with an alkyl group comprising 1 to 20 carbon atoms,
Y is O, S or N, wherein N can be substituted by a group R", wherein R" independently of R and R' has the same meaning as specified for R, R',
Z is $-O^-$, $S^-$, $N(R''')^-$, $N(SO_2R''')^-$, wherein R''' independently of R, R' and R" has the same meaning as specified for R, R', R", or a carbon atom which is part of an aromatic group comprising 1 to 20 carbon atoms,
wherein in case that Y is N, Y and X can be connected within a (hetero)aliphatic or (hetero)aromatic ring having 5 to 6 carbon atoms and/or heteroatoms selected from the group N, P, O and/or S and optionally comprising an annulated aromatic ring having 6 carbon atoms optionally substituted with one or more heteroatom containing groups, wherein the heteroatom is selected from N, O, S, P, Si, F and/or Cl, or with an alkyl group comprising 1 to 20 carbon atoms.

Examples of compounds comprising an anionic moiety having the general formula (V) are $CO_3^{2-}$, $RO-CO_2^-$, $RS-CO_2^-$, $RHN-CO_2^-$, $RR'N-CO_2^-$, $CSO_2^{2-}$, $RO-CSO^-$, $RHN-CSO^-$, $RR'N-CSO^-$, $RS-CSO^-$, $COS_2^{2-}$, $RO-CS_2^-$, $RHN-CS_2^-$, $RR'NCS_2^-$, $RR'N-CSO^-$, $CS_3^{2-}$, $RS-CS_2^-$, $RO-C(=NR'')O^-$, RO-C(=NR")S-, $RS-C(=NR'')O^-$, $RS-C(=NR'')S^-$, $R''N=C(NHR)O^-$, $R''N=C(NRR')O^-$, $R''N=C(NHR)S^-$, $R''N=C(NRR')S^-$, $RO-CO-N(R''')^-$, $RS-CO-N(R''')^-$, $RR'N-CO-N(R''')^-$, $RO-CS-N(R''')^-$, $RS-CS-N(R''')^-$, $RR'N-CS-N(R''')^-$, $RO-C(=NR'')-N(R''')^-$, $RS-C(=NR'')-N(R''')^-$, $RR'N-C(=NR'')-N(R''')^-$, $RO-CO-N(SO_2R''')^-$, $RS-CO-N(SO_2R''')^-$, $RR'N-CO-N(SO_2R''')^-$, $RO-CS-N(SO_2R''')^-$, $RS-CS-N(SO_2R''')^-$, $RR'N-CS-N(SO_2R''')^-$, $RO-C(=NR'')-N(SO_2R''')^-$, $RS-C(=NR'')-N(SO_2R''')^-$, $RR'N-C(=NR'')-N(SO_2R''')^-$,
wherein R, R', R", R''' independently of one another are linear or branched, saturated, mono- or polyunsaturated aliphatic, cycloaliphatic or aromatic groups comprising 1 to 20 carbon atoms, optionally substituted with one or more heteroatom containing groups, wherein the heteroatoms are selected from N, O, S, P, Si, F and/or Cl,
wherein R and R' and/or R''', and/or R' and R" and/or R''' can be connected within a (hetero)aliphatic or (hetero)aromatic, mono- or polycyclic ring system comprising 1 to 20 carbon atoms and/or heteroatoms selected from the group N, P, O and/or S, optionally substituted with one or more heteroatom containing groups, wherein the heteroatom is selected from N, O, S, P, Si, F and/or Cl, or with an alkyl group comprising 1 to 20 carbon atoms, 4,5-dihydro-oxazol-2-olate, optionally substituted in 4- and/or 5-position with an aliphatic, cycloaliphatic or aromatic group having 1 to 20 carbon atoms, oxazol-2-olate, optionally substituted in 4- and/or 5-position with an aliphatic, cycloaliphatic or aromatic group having 1 to 20 carbon atoms, benzoxazol-2-olate, wherein the benzene ring is optionally substituted with one to four aliphatic, cycloaliphatic or aromatic groups having 1 to 20 carbon atoms, 4,5-dihydro-thiazol-2-olate, optionally substituted in 4- and/or 5-position with an aliphatic, cycloaliphatic or aromatic group having 1 to 20 carbon atoms, thiazol-2-olate, optionally substituted in 4- and/or 5-position with an aliphatic, cycloaliphatic or aromatic group having 1 to 20 carbon atoms, benzothiazol-2-olate, wherein the aromatic ring is optionally substituted with one to four aliphatic, cycloaliphatic or aromatic groups having 1 to 20 carbon atoms, 4,5-dihydro-1,3-diazol-2-olate, optionally substituted in 4- and/or 5-position with an aliphatic, cycloaliphatic or aromatic group having 1 to 20 carbon atoms, 1,3-diazol-2-olate, optionally substituted in 4- and/or 5-position with an aliphatic, cycloaliphatic or aromatic group having 1 to 20 carbon atoms, benzo-1,3-diazol-2-olate, wherein the aromatic ring is optionally substituted with one to four aliphatic, cycloaliphatic or aromatic groups having 1 to 20 carbon atoms, 4,5-dihydro-oxazol-2-thiolate, optionally substituted in 4- and/or 5-position with an aliphatic, cycloaliphatic or aromatic group having 1 to 20 carbon atoms, oxazol-2-thiolate, optionally substituted in 4- and/or 5-position with an aliphatic, cycloaliphatic or aromatic group having 1 to 20 carbon atoms, benzoxazol-2-thiolate, wherein the aromatic moiety is optionally substituted with one to four aliphatic, cycloaliphatic or aromatic groups having 1 to 20 carbon atoms, 4,5-dihydro-thiazol-2-thiolate, optionally substituted in 4- and/or 5-position with an aliphatic, cycloaliphatic or aromatic group having 1 to 20 carbon atoms, thiazol-2-thiolate, optionally substituted in 4- and/or 5-position with an aliphatic, cycloaliphatic or aromatic group having 1 to 20 carbon atoms, benzothiazol-2-thiolate, wherein the aromatic moiety is optionally substituted with one to four aliphatic, cycloaliphatic or aromatic groups having 1 to 20 carbon atoms, 4,5-dihydro-1,3-diazol-2-thiolate, optionally substituted in 4- and/or 5-position with an aliphatic, cycloaliphatic or aromatic group having 1 to 20 carbon atoms, 1,3-diazol-2-thiolate, optionally substituted in 4- and/or 5-position with an aliphatic, cycloaliphatic or aromatic group having 1 to 20 carbon atoms, benzo-1,3-diazol-2-thiolate, wherein the aromatic moiety is optionally substituted with one to four aliphatic, cycloaliphatic or aromatic groups having 1 to 20 carbon atoms, pyrimidin-2-yl amide, optionally *N*-substitued, e.g. with aryl or alkylsulfonyl groups, 2-pyrimidin-2-olate, *N*-alkylsulfonyl or *N*-arylsulfonyl carbamimidoylamide, optionally with further *N*'- and/or *N*"-substituents, or $NO_3^-$.

In another embodiment of the invention, the anionic moiety having the general formula (V) comprises $CO_3^{2-}$,

$CSO_2^{2-}$, $COS_2^{2-}$, $RO-CO_2^-$, wherein R is methyl, ethyl, propyl, isopropyl, butyl or phenyl, $RS-CO_2^-$, wherein R is methyl, ethyl, dodecyl or phenyl, $R_2N-CO_2^-$, wherein R is methyl, ethyl or phenyl, $R_2N-CSO^-$, wherein R is methyl, ethyl or phenyl, $R_2N-CS_2^-$, wherein R is methyl, ethyl, butyl or phenyl, $RO-CS_2^-$, wherein R is methyl, ethyl, propyl, isopropyl, butyl or phenyl, 4,5-dihydro-oxazol-2-thiolate, oxazol-2-thiolate, benzoxazol-2-thiolate, 4,5-dihydro-thiazol-2-thiolate, thiazol-2-thiolate, benzothiazol-2-thiolate, 4,5-dihydro-1,3-diazol-2-thiolate, 1,3-diazol-2-thiolate, benzo-1,3-diazol-2-thiolate or $NO_3^-$.

In a preferred embodiment of the invention, the anionic moiety of the catalyst having the general formula (V) is selected from the group comprising $CO_3^{2-}$, $NO_3^-$, $CSO_2^{2-}$, $COS_2^{2-}$, $(CH_3)_2N-CS_2^-$, $(C_2H_5)_2N-CS_2^-$, and benzothiazol-2-thiolate.

In another preferred embodiment of the invention, the anionic moiety of the catalyst having the general formula (V) is selected from the group comprising $CO_3^{2-}$, $NO_3^-$, $(CH_3)_2N-CS_2^-$ or $(C_2H_5)_2N-CS_2^-$.

In another embodiment of the invention, the catalyst comprises two or more different anionic moieties of the general formula (V) as a mixture of separate compounds or combined in one common larger molecular framework.

In another embodiment of the invention, the conjugated trigonal-planar anionic moiety comprised in the catalyst is generated from an appropriate nucleophile and carbon dioxide $CO_2$, carbonyl sulphide SCO or carbon disulfide $CS_2$. Appropriate nucleophiles can be primary, secondary or tertiary alcohols, such as methanol, ethanol, n-propanol, isopropanol, *n*-butanol, isobutanol, *tert*-butanol, the different pentanol isomers, the different hexanol isomers and higher homologues, cyclopentanol, cyclohexanol, aromatic alcohols such as phenol or napththol, which may be further substituted, but preferably carry hydrogen atoms in 2-position to the hydroxyl group, primary, secondary or tertiary thiols, such as methane thiol, ethane thiol, n-propane thiol, isopropane thiol, *n*-butane thiol, isobutane thiol, *tert*-butane thiol, the different pentane thiol isomers, the different hexane thiol isomers and higher homologues, cyclopentane thiol, cyclohexane thiol, aromatic thiols such as thiophenol or thionapththol, which may be further substituted, but preferably carry hydrogen atoms in 2-position to the thiol group, monoalkyl amines such as methyl amine, ethyl amine, *n*-propyl amine, isopropyl amine, butyl amine and their higher homologues, cyclohexylamine, dialkylamines such as dimethylamine, diethylamine, di-*n*-propyl amine, diisopropyl amine, and their higher homologues, aromatic amines such as aniline, naphthyl amine and diphenyl amine, wherein the aromatic substituents may be further substituted, but preferably carry hydrogen atoms in 2-position to the amino group, *N*-alkyl-*N*-aryl amines such as *N*-methyl aniline, *N*-ethyl aniline and their higher homologues, ethanol amine or *N*-substituted ethanol amines. In the case of the above mentioned nucleophiles, especially alcohols and thiols, a non-nucleophilic base may be added during the generation of the anionic moiety. Further appropriate nucleophiles are primary, secondary or tertiary alcoholates or thiolates derived from the primary, secondary or tertiary alcohols or thiols listed above by deprotonation with an appropriate base such as metal hydrides, e.g. sodium hydride or potassium hydride, or alkaline metals such as sodium or potassium. Further appropriate nucleophiles are amides derived by deprotonation from any type of monoalkyl amine, dialkyl amine, aromatic amine or *N*-alkyl-*N*-aryl amine specified above, such as lithium diisopropylamide, hexamethyldisilazane. The generation of the anionic moiety from an appropriate nucleophile and carbon dioxide, carbonyl sulphide or carbon disulphide can be performed *ex situ,* i.e. in a separate reaction vessel, and then be injected into the reaction mixture, or *in situ,* meaning that the precursors (nucleophile and carbon dioxide or carbonyl sulphide or carbon disulphide) are added to the reaction vessel, either before or after addition of one or more of the substrates. Preferred nucleophiles are alcoholates such as sodium or potassium methylate, sodium or potassium ethylate, sodium or potassium *n*- or isopropylate, or higher linear or branched aliphatic or cycloaliphatic homologues, dialkylamines such as dimethylamine, diethylamine, di-*n*-propylamine, diisopropylamine and dialkylamines with larger linear or branched aliphatic or cycloaliphatic alkyl-substituents.

An appropriate cation in the catalyst can be a Lewis acidic cation.

In one embodiment of the invention, the catalyst according to the invention is a salt comprising a conjugated trigonal-planar anionic moiety, preferably an anionic moiety according to formula (V), and a cationic Lewis acid as counter cation. The cationic Lewis acid can be a metal cation or a cationic metal complex comprising a metal selected from the group comprising Li(I), Rb(I), Cs(I), Ag(I), Au(I), Mg(II), Ca(II), Sr(II), Ba(II), Dy(II), Yb(II), Cu(II), Zn(II), V(II), Mo(II), Mn(II), Fe(II), Co(II) Ni(II), Pd(II), Pt(II), Ge(II), Sn(II), Sc(III), Y(III), La(III), Ce(III), Pr(III), Nd(III), Sm(III), Eu(III), Gd(III), Tb(III), Dy(III), Ho(III), Er(III), Tm(III), Yb(III), Lu(III), Hf(III), Nb(III), Ta(III), Cr(III), Ru(III), Os(III), Rh(III), Ir(III), Al(III), Ga(III), In(III), Tl(III), Ge(III), Ce(IV), Ti(IV), Zr(IV), Hf(IV), Nb(IV), Mo(IV), W(IV), Ir(IV), Pt(IV), Sn(IV), Pb(IV), Nb(V), Ta(V), P(V), Sb(V), Bi(V), Mo(VI), W(VI).

In the cationic metal complex, an appropriately chosen ligand, such as phosphine, phosphite, arsenite, alkyl, acetylacetonate may be coordinated to the vacant coordination sites at the metal, whereby at least one coordination site remains available for binding of a Lewis base. The coordination site for binding of a Lewis base may also be created in the reaction mixture, e.g., by dissociation of a weakly coordinating ligand.

In one preferred embodiment of the invention, the cationic part of the catalyst is represented by formula (VI)

$$[M(R1)(R2)(R3)(R4)]^+ \qquad (VI)$$

wherein

M is phosphorous or antimony,
(R1), (R2), (R3), (R4) are independently of one another selected from the group comprising linear or branched alkyl groups containing 1 to 22 carbon atoms, optionally substituted with heteroatoms and/or heteroatom containing substituents, cycloaliphatic groups containing 3 to 22 carbon atoms, optionally substituted with heteroatoms and/or heteroatom containing substituents, C1 to C3 alkyl-bridged cycloaliphatic groups containing 3 to 22 carbon atoms, optionally substituted with heteroatoms and/or heteroatom containing substituents and aryl groups containing 6 to 18 carbon atoms, optionally substituted with alkyl groups containing 1 to 10 carbon atoms and/or heteroatoms.

In another preferred embodiment of the invention, a catalyst as described above is used wherein the cationic part of the catalyst having formula (VI) is used, wherein
M is phosphorous or antimony,
(R1), (R2), (R3), (R4) are independently of one another selected from the group comprising phenyl, *n*-butyl and cyclohexyl.

In one preferred embodiment of the invention the catalyst according to claim 1 for the reaction of isocyanates with epoxides or reaction of diisocyanates with diepoxides comprises at least one anionic moiety of formula (V) and at least one cationic moiety of formula (VI) as described before.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$, $NO_3^-$, $CSO_2^{2-}$, $COS_2^{2-}$, $(CH_3)_2N\text{-}CS_2^-$, $(C_2H_5)_2N\text{-}CS_2^-$ and benzothiazol-2-thiolate and at least one cationic moiety of formula (VI), wherein M is phosphorous or antimony, (R1), (R2), (R3), (R4) are independently of one another selected from the group comprising phenyl, *n*-butyl and cyclohexyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$, $NO_3^-$, $CSO_2^{2-}$, $COS_2^{2-}$, $(CH_3)_2N\text{-}CS_2^-$, $(C_2H_5)_2N\text{-}CS_2^-$ and benzothiazol-2-thiolate and at least one cationic moiety of formula (VI), wherein M is phosphorous or antimony, and (R1), (R2), (R3), (R4) are equal and are selected from the group comprising phenyl, *n*-butyl and cyclohexyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$, $NO_3^-$, $CSO_2^{2-}$, $COS_2^{2-}$, $(CH_3)_2N\text{-}CS_2^-$, $(C_2H_5)_2N\text{-}CS_2^-$ and benzothiazol-2-thiolate and at least one cationic moiety of formula (VI), wherein M is phosphorous, and (R1), (R2), (R3), (R4) are equal and are selected from the group comprising phenyl, *n*-butyl and cyclohexyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$, $NO_3^-$, $CSO_2^{2-}$, $COS_2^{2-}$, $(CH_3)_2N\text{-}CS_2^-$, $(C_2H_5)_2N\text{-}CS_2^-$ and benzothiazol-2-thiolate and at least one cationic moiety of formula (VI), wherein M is phosphorous, and (R1), (R2), (R3), (R4) are all phenyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$, $NO_3^-$, $CSO_2^{2-}$, $COS_2^{2-}$, $(CH_3)_2N\text{-}CS_2^-$, $(C_2H_5)_2N\text{-}CS_2^-$ and benzothiazol-2-thiolate and at least one cationic moiety of formula (VI), wherein M is phosphorous, and (R1), (R2), (R3), (R4) are all *n*-butyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$, $NO_3^-$, $CSO_2^{2-}$, $COS_2^{2-}$, $(CH_3)_2N\text{-}CS_2^-$, $(C_2H_5)_2N\text{-}CS_2^-$ and benzothiazol-2-thiolate and at least one cationic moiety of formula (VI), wherein M is phosphorous, and (R1), (R2), (R3), (R4) are all cyclohexyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$, $NO_3^-$, and at least one cationic moiety of formula (VI), wherein M is phosphorous, and (R1), (R2), (R3), (R4) are equal and are selected from the group comprising phenyl, *n*-butyl and cyclohexyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$ and $NO_3^-$, and at least one cationic moiety of formula (VI), wherein M is phosphorous, and (R1), (R2), (R3), (R4) are equal and phenyl.

In another preferred embodiment of the invention, the catalyst according to claim 1 for the reaction of isocyanates with epoxides or reaction of diisocyanates with diepoxides comprises at least one anionic moiety selected from the group comprising $CO_3^{2-}$ and $NO_3^-$, and at least one cationic moiety of formula (VI), wherein M is phosphorous, and (R1), (R2), (R3), (R4) are equal and *n*-butyl.

In another preferred embodiment of the invention, the catalyst according to claim 1 for the reaction of isocyanates with epoxides or reaction of diisocyanates with diepoxides comprises at least one anionic moiety selected from the group

comprising $CO_3^{2-}$ and $NO_3^-$, and at least one cationic moiety of formula (VI), wherein M is phosphorous, and (R1), (R2), (R3), (R4) are equal and cyclohexyl.

In another preferred embodiment of the invention, the catalyst according to claim 1 for the reaction of isocyanates with epoxides or reaction of diisocyanates with diepoxides comprises $CO_3^{2-}$ and at least one cationic moiety of formula (VI), wherein M is phosphorous or antimony, (R1), (R2), (R3), (R4) are independently of one another selected from the group comprising phenyl, *n*-butyl and cyclohexyl.

In another preferred embodiment of the invention, the catalyst according to claim 1 for the reaction of isocyanates with epoxides or reaction of diisocyanates with diepoxides comprises $CO_3^{2-}$ and at least one cationic moiety of formula (VI), wherein M is phosphorous, (R1), (R2), (R3), (R4) are independently of one another selected from the group comprising phenyl, *n*-butyl and cyclohexyl.

In another preferred embodiment of the invention, the catalyst according to claim 1 for the reaction of isocyanates with epoxides or reaction of diisocyanates with diepoxides comprises $CO_3^{2-}$ and at least one cationic moiety of formula (VI), wherein M is phosphorous, (R1), (R2), (R3), (R4) are equal and selected from the group comprising phenyl, *n*-butyl and cyclohexyl.

In another preferred embodiment of the invention, the catalyst according to claim 1 for the reaction of isocyanates with epoxides or reaction of diisocyanates with diepoxides comprises $CO_3^{2-}$ and at least one cationic moiety of formula (VI), wherein M is phosphorous, (R1), (R2), (R3), (R4) are equal and phenyl.

In another preferred embodiment of the invention, the catalyst according to claim 1 for the reaction of isocyanates with epoxides or reaction of diisocyanates with diepoxides comprises $CO_3^{2-}$ and at least one cationic moiety of formula (VI), wherein M is phosphorous, (R1), (R2), (R3), (R4) are equal and *n*-butyl.

In another preferred embodiment of the invention, the catalyst according to claim 1 for the reaction of isocyanates with epoxides or reaction of diisocyanates with diepoxides comprises $CO_3^{2-}$ and at least one cationic moiety of formula (VI), wherein M is phosphorous, (R1), (R2), (R3), (R4) are equal and cyclohexyl.

In another embodiment of the invention in combination with one or more of the aforementioned embodiments the isocyanate compound is selected from benzyl isocyanate, phenyl isocyanate, *ortho-, meta-, para*-tolyl isocyanate, dimethylphenyl isocyanate (technical mixture and individual isomers), 4-cyclohexylphenyl isocyanate and/or *ortho-, meta-, para*-methoxyphenyl isocyanate and the epoxide compound is selected from ethylene oxide, propylene oxide, 1,2-butene oxide, 2,3-butene oxide, styrene oxide, butyl glycidyl ether, benzyl glycidyl ether, phenyl glycidyl ether, p-tolyl glycidyl ether and/or 4-tert-butylphenyl glycidyl ether.

In another embodiment of the invention in combination with one or more of the aforementioned embodiments the diisocyanate compound is selected from hexamethylene diisocyanate (HDI), 3-isocyanatomethyl-3,3,5-trimethylcyclohexyl isocyanate (isophorone diisocyanate, IPDI), 4,4'-diisocyanatodicyclohexylmethane ($H_{12}$-MDI), 2,4- or 2,5- or 2,6-diisocyanatotoluene (TDI) or mixtures of these isomers and/or 4,4'-, 2,4'- or 2,2'-diisocyanatodiphenylmethane (MDI) or mixtures of these isomers and the diepoxide compound is selected from butadiene diepoxide, the diepoxides of double unsaturated fatty acid C1 - C18 alkyl esters, ethylene glycol diglycidyl ether, di(ethylene glycol) diglycidyl ether, poly(ethylene glycol) diglycidyl ether, propylene glycol diglycidyl ether, di(propylene glycol) diglycidyl ether, poly(propylene glycol) diglycidyl ether, neopentyl glycol diglycidyl ether, 1,6-hexanediol diglycidyl ether, hydrogenated bisphenol-A diglycidyl ether, 1,2-dihydroxybenzene diglycidyl ether, resorcinol diglycidyl ether, 1,4-dihydroxybenzene diglycidyl ether, bisphenol-A diglycidyl ether, diglycidyl o-phthalate, diglycidyl isophthalate and/or diglycidyl terephthalate.

In one embodiment the method according to the invention is performed in the presence of a solvent. Suitable solvents are high-boiling non-protic solvents such as *N*-methylpyrrolidone (NMP), *N*-ethylpyrrolidone, cyclic ethylene carbonate, cyclic propylene carbonate, sulfolane, chlorobenzene, the different isomers of dichlorobenzene, the different isomers of trichlorobenzene, decalin, hexamethylphosphoramide, dimethylformamide, N,N-dimethylacetamide (DMAc), dimethyl sulfoxide or mixtures of one or more of the aforementioned solvents among each other or with further non-protic solvents. Preferred solvents are NMP, sulfolane and/or DMAc.

In another embodiment of the method according to the invention, the reaction is conducted in the absence of a solvent. Preferably, the reaction mixture contains only the epoxide compound(s), the isocyanate compound(s) and the catalyst(s), as well as the oxazolidinone compound formed during the reaction.

The isocyanate compound may be added to the epoxide compound in a continuous or step-wise manner with two or more individual addition steps in the step-wise addition wherein in each individual addition step the amount of isocyanate compound added is $\leq$ 50 weight-% of the total amount of isocyanate compound to be added. This is to be understood in such a way that during the course of the reaction the isocyanate compound is added to the reaction mixture containing the epoxide compound continuously or in the aforementioned step-wise manner. Included is also the case that the isocyanate compound is added *via* a syringe pump, dripping funnel or other continuous or semi-continuous devices where the isocyanate is brought into the reaction mixture. Although some after-reaction time may be given to the reaction system, the reaction should be essentially complete shortly after the end of the addition of the isocyanate compound.

By way of process criteria, one could establish a condition that 30 minutes, preferably 20 minutes and more

preferred 10 minutes after the end of the isocyanate addition no more change in the NCO group content of the reaction mixture takes place (within the boundaries of experimental uncertainty). This may be observed, for example, by *in-situ* IR spectroscopy or analysis of samples of the reaction mixture concerning their NCO content, for example, by titration according to DIN ISO 10283.

In one embodiment of the method according to the invention, the isocyanate compound is added continuously to the reaction mixture. "Continuously" in the meaning of the invention means that the isocyanate compound is added to the reaction mixture over a defined period of time, while at the same time any isocyanate compound present in the reaction mixture is converted to the oxazolidinone compound. Preferably, the rate of isocyanate addition is smaller than or equal to the maximum rate, with which the isocyanate compound can be converted under these reaction conditions to the oxazolidinone compound in order to avoid accumulation of NCO groups in the reaction mixture. The actual concentration of NCO groups in the reaction mixture may be observed, for example, by *in-situ* IR spectroscopy. If the NCO group concentration is observed to increase above a set value, the rate of isocyanate addition is reduced. Preferably, the isocyanate compound is added to the reaction mixture (consisting of epoxide compound, isocyanate compound, onium salt and oxazolidinone compound, but not considering solvent, if present) with such an addition rate that the concentration of the isocyanate compound in the reaction mixture is ≤ 40 weight-%, preferably ≤ 20 weight-% and more preferred < 15 weight-%.

In another embodiment of the method according to the invention, in each individual addition step the amount of isocyanate compound added is ≥ 0.1 weight-% to ≤ 50 weight-% of the total amount of isocyanate compound to be added. Preferably, the amount of isocyanate compound added per individual addition step is ≥ 1.0 weight-% to ≤ 40 weight-% of, more preferred ≥ 5.0 weight-% to ≤ 35 weight-% of the total amount of isocyanate compound to be added. Preferably, the time intervals between each individual addition of isocyanate compound to the reaction mixture (consisting of epoxide compound, isocyanate compound, onium salt and oxazolidinone compound, but not considering solvent, if present) is chosen in such a way that the concentration of the isocyanate compound in the reaction mixture at any given point in time is ≤ 40 weight-%, preferably ≤ 20 weight-% and more preferred ≤ 15 weight-%. The actual concentration of NCO groups in the reaction mixture may be observed, for example, by *in-situ* IR spectroscopy. If the NCO group concentration is observed to increase above a set value, the time interval between the addition steps is increased.

In another embodiment of the method according to the invention, the isocyanate compound is a polyisocyanate compound with two or more NCO groups per molecule, preferably with two NCO groups per molecule and the epoxide compound is a polyepoxide compound with two or more epoxy groups per molecule, preferably with two epoxy groups per molecule.

In another embodiment of the invention the catalyst is present in an amount of ≥ 0.0001 mol-% to ≤ 4.0 mol-%, based on the initial amount of epoxide compound. Preferably, the catalyst is present in an amount of ≥ 0.001 mol-% to ≤ 2.0 mol-%, more preferred > 0.01 mol-% to ≤ 1.1 mol-%.

The method according to the invention is performed for example at temperatures of ≥ 130 °C to ≤ 280 °C, preferably at a temperature of ≥ 135 °C to ≤ 240 °C, most preferred at a temperature of ≥140°C to ≤ 210°C.

In another embodiment of the method according to the invention, the oxazolidinone product is obtained with a chemoselectivity $S_{OXA}$, given as the ratio of the molar amount of oxazolidinone moieties to the sum of the molar amount of oxazolidinone moieties and the molar amount of isocyanurate moieties, of > 93 %, preferably > 96 % and most preferably > 99 %. The determination of the chemoselectivity $S_{OXA}$ is described below in the methods.

The present invention is further directed towards an oligomeric or polymeric oxazolidinone compound, obtainable by a method according to the invention using an isocyanate compound with at least two NCO groups per molecule, preferably two NCO groups per molecule, and an epoxide compound with at least two epoxy groups per molecule, preferably two epoxy groups per molecule, comprising at least one unit derived from the isocyanate compound and at least two units derived from the epoxide compound or comprising at least one unit derived from the epoxide compound and at least two units derived from the isocyanate compound. The colour of these oligomeric or polymeric oxazolidinone compounds is < 21 CVI, preferably < 15 CVI and more preferably < 11 CVI in reference to the iodine colour scale (DIN6162). The determination of the colour is described in the methods.

The chain length for the prepolymers can be controlled by changing the ratio between the diepoxide and the diisocyanate compound. An epoxide terminated oligomer is obtained, when the diepoxide is employed in excess. An isocyanate terminated oligomer is obtained, when the diisocyanate is employed in excess. Linear polymer chains with high molecular weight are obtained, when equimolar amounts of diepoxide and diisocyanate are reacted with each other. The precise content of epoxy and isocyanate groups in the diepoxide and diisocyanate, respectively, are preferentially determined before the polymerisation reaction, e.g., by measuring the epoxide number according to German standard norm DIN EN 1877-1 and the isocyanate number according to German standard norm DIN EN ISO 11909.

The number average molecular weight $M_n$ of the polyoxazolidinone compound may be, for example, in the range of ≥ 320 g/mol to ≤ 10'000'000 g/mol, preferably ≥ 480 g/mol to ≤ 600'000 g/mol and most preferably > 3'000 g/mol to ≤ 200'000 g/mol. The number average molecular weight can be calculated by end-group analysis based on $^1$H NMR spectroscopy or determined by gel permeation chromatography (GPC), as described in detail in the methods below.

Without being bound to a theory molecular weight is determined by the molar ratio of polyisocyanate compound to polyepoxide compound. An alternative method to control the molecular weight of the oligooxazolidinone or polyoxazolidinone product constitutes in adding a small amount of a monoepoxide or monoisocyanate to the mixture of polyepoxide and the polyisocyanate compound.

The polyoxazolidinone compounds obtained by a method according to the invention may have a glass transition temperature Tg of >155 °C, preferably ≥160 °C, most preferably ≥169 °C. The glass transition temperature Tg can be determined by differential scanning calorimetry (DSC) as described below in the methods.

Preferably this oligomeric or polymeric oxazolidinone compound comprises at at least one, preferably at least two and particularly preferred two terminal epoxide and/or isocyanate groups.

It is also possible that this oligomeric or polymeric oxazolidinone compound comprises at least one terminal group which is non-reactive towards epoxide and/or isocyanate groups. Examples include alkoxy groups or trialkylsiloxane groups.

The invention is further directed to the use of a catalyst in the reaction of isocyanate compounds or polyisocyanate compounds with epoxide compounds or polyepoxide compounds, wherein the catalyst is free of halide anions, and comprises a conjugated trigonal-planar anionic moiety having a central atom selected from the elements C or N and three further substituent atoms, selected independently of one another from the elements C, N, O, S and/or P, and wherein the overall charge state of the conjugated trigonal-planar anionic moiety is -1 or -2.

The method according to the invention is suited for the synthesis of oxazolidinone compounds with interesting properties for use, for example, as pharmaceutics or antimicrobiotics. Polyoxazolidinones obtained by the method according to the invention are particularly suited as polymer building blocks in polyurethane chemistry. For example, epoxy-terminated oligomeric oxazolidinones (oligooxazolidinones) may be reacted with polyols or polyamines to form foams or thermosets. Such epoxy-terminated oligomeric oxazolidinones are also suited for the preparation of composite materials. Epoxy-terminated oligomeric oxazolidinones (oligooxazolidinones) may also be reacted with their NCO-terminated counterparts to form high molecular weight polyoxazolidinones, which are useful as transparent, high temperature-stable materials. Polyoxazolidinones with high molecular weight obtained by the method according to the invention are particularly suited as transparent, high temperature-stable thermoplastic materials.

## Examples

*Epoxides*

Monoepoxide 1: *R*-(+)-Styrene oxide, purity 99 %; stored under argon at -4 °C. Acros Organics, Belgium.

Diepoxide 1: Bisphenol-A-diglycidylether (Epikote™ Resin 162), purity +99 %, stored at room temperature, Momentive, Germany.

*Isocyanates*

Monoisocyanate 1: *para*-Tolyl isocyanate, purity 99 %; distilled in a partial vacuum (200 mbar, 80 °C) and stored under argon at -4 °C prior to use, Acros Organics, Belgium.

Monoisocyanate 2: *para*-Pentylphenyl isocyanate, purity > 99 %; obtained by reaction of *para*-pentylaniline with phosgene.

Diisocyanate 1: 2,4-Toluenediisoyanate, >99% 2,4-isomer, Bayer MaterialScience AG, Germany

*Solvents:*

If not mentioned otherwise, solvents were purchased from Sigma-Aldrich, Germany, and used as received.

Sulfolane, purity 99%, was obtained from Sigma-Aldrich, Germany.

*N*-Methylpyrrolidinone (NMP), purity 99.5 %, anhydrous, was obtained from Sigma-Aldrich, Germany.

*Catalysts*

Catalyst 1: Bis-tetraphenylphosphonium carbonate

A mixture of silver carbonate (0.5 g, 1.81 mmol) and methanol (100 mL) was stirred under complete darkness for 1 h at 25 °C, whereupon a partial dissolution of the solid occurred. The clear, overstanding solution was then added dropwise to a solution of tetraphenylphosphonium bromide (1.52 g, 3.62 mmol) in methanol (4 mL) and the resulting mixture stirred in darkness at 25 °C for 3 h. After resting for 16 h, a yellow precipitate had formed, which was removed by filtration. The volatile components of the filtrated solution were then removed by evaporation under reduced pressure

to give 1.45 g of a colourless solid.

Catalyst 2: Tetraphenylphosphonium nitrate

A solution of 0.81 g (4.76 mmol) silver nitrate in 33 mL ethanol was prepared at 50 °C and added dropwise to a solution of 1.0 g (2.38 mmol, 0.5 eq.) tetraphenylphosphonium bromide in 2 mL ethanol. The resulting yellow precipitate was removed by filtration. The volatile components of the filtrated solution were removed by evaporation under reduced pressure to give 0.39 g of a colourless solid.

Catalyst 3 (Comp.): Tetraphenylphosphonium chloride 98 % , Sigma-Aldrich, Germany.
Catalyst 4 (Comp.): Tetraphenylphosphonium bromide 97 %, Sigma-Aldrich, Germany.

*Reactor:*

The 300 ml stainless steel autoclave used in the synthesis of polyoxazolidinones had a height (internal) of 10.16 cm and an internal diameter of 6.35 cm. The reactor was fitted with an electric heating jacket (510 watt maximum heating capacity). The counter cooling consisted of a U-shaped dip tube with an external diameter of 6 mm, which projected into the reactor to within 5 mm of the bottom and through which cooling water at approximately 10°C was passed. The water stream was switched on and off by means of a solenoid valve. The reactor was also fitted with an inlet tube and a temperature probe of 1.6 mm diameter, both of which projected into the reactor to within 3 mm of the bottom. The impeller agitator used in the examples was a pitched-blade turbine to which a total of two agitator stages, each with four agitator blades (45°) of 35 mm diameter and 10 mm height, were attached to the agitator shaft at a distance of 7 mm.

In the terminology used in the examples, the term "pulse addition", corresponding to the step-wise addition mode of the isocyanate, means that the isocyanate compound is added in individual steps, comprising a part of the total amount of the isocyanate compound, to the reaction mixture.

*In-situ infrared (IR) spectroscopy:*

The composition of the reaction mixture during the synthesis of oxazolidinone 1 was followed with a Matrix-MF spectrometer (Bruker Optic GmbH) equipped with a high-pressure attenuated total reflectance (ATR) IR fibre optical probe. The ATR IR fibre optical probe (3.17 mm outer diameter, 90° diamond prism with 1 $\times$ 2 mm basal area and 1 mm height as ATR element, 2 $\times$ 45° reflection of the IR beam, IR beam coupled *via* fibre optics) was fitted into the reactor in such a way that the diamond at the end of the optical probe was immersed entirely into the reaction mixture. The IR spectra were recorded using the OPUS 6.5 software. Before starting the experiment, a background spectrum was taken against ambient air (scan velocity 40 kHz, average of 100 scans). During the experiment, Infrared spectra were recorded every 15 seconds (scan velocity 40 kHz, average of 20 scans). Complete conversion was indicated by the disappearance of the characteristic isocyanate band at 2250-2260 $cm^{-1}$. The formation of oxazolidinone moieties was indicated by the appearance of the characteristic oxazolidinone band at 1743 $cm^{-1}$. The formation of isocyanurate moieties was indicated by the appearance of the characteristic isocyanurate band at 1690 $cm^{-1}$.

The spectra were analysed with the software PEAXACT 3.1 - Software for Quantitative Spectroscopy and Chromatography, S•PACT GmbH using the Integrated Hard Model (IHM) method. The hard model for analysing the IR spectra of the reaction mixtures was created by linear combination of hard models for the single components monoisocyanate, oxazolidinone and isocyanurate. The hard model for the single components was created as follows:

Monoisocyanate 1:

A 10 mL glass flask containing 5 mL propylene carbonate was immersed into an oil bath preheated to 160 °C. A background IR spectrum (average of 100 scans) was measured against air. The IR probe was then inserted into the glass flask in such a way that the diamond at the end of the optical probe was immersed entirely into the reaction mixture. After flushing the glass flask with argon, the glass flask was capped with a rubber seal and another background IR spectrum was measured (average of 100 scans). Subsequently, monoisocyanate 1 (0.37 mL, 2.95 mmol, 5 mol% relative to propylene carbonate) was injected with a microliter syringe through the seal into the reaction mixture and several IR spectra were recorded of the resulting mixture. The relevant signals in the region 2150 to 2400 $cm^{-1}$ were selected from a representative IR spectrum and the model refined to achieve an optimum fit between a superposition of the selected signals and the averaged IR spectra of the mixture.

Oxazolidinone 1:

A 10 mL glass flask containing 0.5 mL propylene carbonate was immersed into an oil bath preheated to 160 °C. A background IR spectrum (average of 100 scans) was measured against air. The IR probe was then inserted into the glass flask in such a way that the diamond at the end of the optical probe was immersed entirely into the reaction mixture. After flushing the glass flask with argon, the glass flask was capped with a rubber seal and another background IR spectrum was measured (average of 100 scans). Subsequently, a solution of oxazolidinone 1 (0.471 g, 1.18 mmol, 10 mol% relative to propylene carbonate) in 0.5 mL propylene carbonate was injected with a microliter syringe through the seal into the reaction mixture and several IR spectra were recorded of the resulting mixture. The relevant signals in the region 1650 to 1800 $cm^{-1}$ were selected from a representative IR spectrum and the model refined to achieve an optimum fit between a superposition of the selected signals and the averaged IR spectra of the mixture.

Isocyanurate 1:

A 10 mL glass flask containing 0.5 mL propylene carbonate was immersed into an oil bath preheated to 160 °C. A background IR spectrum (average of 100 scans) was measured against air. The IR probe was then inserted into the schlenk flask in such a way that the diamond at the end of the optical probe was immersed entirely into the reaction mixture. After flushing the glass flask with argon, the Glass flask was capped with a rubber seal and another background IR spectrum was measured (average of 100 scans). Subsequently, a solution of isocyanurate 1 (0.471 g, 1.18 mmol, 10 mol% relative to propylene carbonate) in 0.5 mL propylene carbonate was injected with a microliter syringe through the seal into the reaction mixture and several IR spectra were recorded of the resulting mixture. The relevant signals in the region 1600 to 1750 $cm^{-1}$ were selected from a representative IR spectrum and the model refined to achieve an optimum fit between a superposition of the selected signals and the averaged IR spectra of the mixture.

The IR spectra obtained *in-situ* during the catalyst tests were analysed by performing a hard modelling factor analysis (HMFA) as described in E. Kriesten, D. Mayer, F. Alsmeyer, C. B. Minnich, L. Greiner and W. Marquardt, Chemomet. Intell. Lab. Sys., 93(2), 108 (2008) using the hard models obtained by the method described above. The molar amount n; of the respective component i was calculated.

The chemoselectivity $S_{OXA}$ towards oxazolidinone was calculated at the end point of the reaction based on the molar yield of oxazolidinone (OXA) and isocyanurate (ICT) according to eq. 1,

$$S_{oxa} = \frac{n^{fin}{}_{OXA}}{n^{fin}{}_{OXA} + n^{fin}{}_{ICT}} \quad \text{(eq. 1)}$$

where $n^{fin}{}_{OXA}$ is the molar amount of oxazolidinone at the end of the reaction and $n^{fin}{}_{ICT}$ is the molar amount of isocyanurate at the end of the reaction.

The turnover frequency ($TOF_{1/2}$) as measure for reaction rate was determined according to eq. 2,

$$TOF_{1/2} = \frac{n_{NCO,t_0} - n_{NCO,t_{1/2}}}{n_{cat} \times t_{1/2}} \quad \text{(eq. 2)}$$

where $t_{1/2}$ is half the time interval from injection of the second monoisocyanate pulse until no further decrease of the monoisocyanate concentration was observed, $n_{NCO,t0}$ is the molar amount of monoisocyanate in the reaction mixture after injection of the second monoisocyanate pulse, $n_{NCO,t1/2}$ is the residual molar amount of monoisocyanate observed by *in-situ* IR spectroscopy at $t_{1/2}$ after injection of the second monoisocyanate pulse, and $n_{cat}$ is the molar amount of catalyst in the reaction mixture after injection of the second isocyanate pulse.

*Solid state infrared (IR) spectroscopy:*

The solid state IR analysis was performed on a Bruker ALPHA-P IR spectrometer equipped with a diamond probe head. Before obtaining the IR spectrum of the sample, a background spectrum was recorded against ambient air. After that, a small sample (several milligrams) of the prepolymer sample was applied to diamond probe and the IR spectrum recorded on a computer connected with the spectrometer using OPUS 6.5 software averaging over 32 spectra obtained in the range of 4000 to 400 $cm^{-1}$ with a resolution of 4 $cm^{-1}$.

*Colorimetry:*

The colorimetric measurements were performed on a Hach Lange Lico 150 Colorimeter. For the measurement a solution of the sample in *N*,*N*-dimethylacetamide (c = 5 mg/mL) was prepared. For reference pure *N*,*N*-dimethylacetamide was used. For oxazolidinone 1, the listed values are given in Hazen units (HU) and refer to the Hazen (Pt/Co) scale (ISO 6271). In case of polyoxazolidinone 1, the listed values refer to the iodine color value (CVI, DIN 6162).

*Gel permeation chromatography (GPC):*

GPC measurements were performed at 40 °C in N,N-dimethylacetamide (DMAc, flow rate of 0.6 mL $min^{-1}$). The column set consisted of 4 consecutive columns (GRAM 3000, HEMA300, HEMA 40, HEMA 40). Samples (concentration 5-15 g $L^{-1}$, injection volume 100 μL) were injected employing an HP1200 auto sampler. A UV-detector was used for following the concentration at the exit of the column. Raw data were processed using the PSS WinGPC Unity software package. Polystyrene of known molecular weight was used as reference to calculate the molecular weight distribution. The number average molecular weight measured by GPC is denominated as $M_n$(GPC) in the examples.

*Thermogravimetric analysis (TGA):*

The stability of the polyoxazolidinones was characterised by thermogravimetric analysis (TGA). The measurements were performed on a Mettler Toledo TGA/DSC 1. The sample (6 to 20 mg) was heated from 25 °C to 600 °C with a heat rate of 10 K/min and the relative weight loss followed in dependence of temperature. For data analysis the software $STAR^e$ SW 11.00 was used. As decomposition temperature the inflection point of the sinuidal weight loss curve is stated.

*Differential scanning calorimetry (DSC):*

The glass transition point $T_g$ was recorded on a Mettler Toledo DSC 1. The sample (4 to 10 mg) was heated from 25 °C to 250 °C at a heating rate of 10 K/min then cooled down to 30 °C at a rate of 10 K/min. This heating cycle was repeated four times. For data analysis the software $STAR^e$ SW 11.00 was used. For determination of the glass transition temperature a tangential analysis method was used. The midpoint of the intersection point between the tangent at low temperature and the tangent in the mid temperature range and the intersection point between the tangent in the mid temperature range and the tangent at high temperature is stated.The reported $T_g$ was taken from the third heating cycle.

*Polymerisation reactions*

General procedure 1: Synthesis of oxazolidinone 1 from monoepoxide 1 and monoisocyanate 1

A 10 mL glass flask containing catalyst, monoepoxide 1 (1 mL, 8.77 mmol), a cylindrical magnetic stirring bar (PTFE, length 10 mm, diameter 6 mm) and optionally 2 mL of solvent was immersed into a hot oil bath set to 160 °C and stirring started. A single IR background spectrum (average of 100 scans) was measured at air, then the IR probe was inserted into the glass flask in such a way that the diamond at the end of the optical probe was immersed entirely into the reaction mixture. After flushing the flask with argon, the glass flask was capped and another single IR background spectrum was measured (average of 100 scans). Then, IR measurement was started. IR spectra (average of 20 scans) were recorded throughout the entire experiment at 60 second time intervals. Subsequently, a pulse of monoisocyanate 1 (0.36 mL, 2.92 mmol, 0.33 equiv. with respect to monoepoxide 1) was injected with a microliter syringe through the seal into the reaction mixture. When the intensity of the monoisocyanate signal in the IR spectrum had decreased below 0.005 absorbance units, a second pulse of monoisocyanate 1 (0.36 mL, 2.92 mmol, 0.33 equiv. with respect to monoepoxide 1) was injected into the reaction mixture. When after injection of the second monoisocyanate pulse the intensity of the monoisocyanate signal in the IR spectrum had decreased below 0.005 absorbance units, a third pulse of monoisocyanate 1 (0.36 mL, 2.92 mmol, 0.33 equiv. with respect to monoepoxide 1) was injected into the reaction mixture. When after injection of the third monoisocyanate pulse monoisocyanate 1 had been completely consumed (indicated by the disappearance of the isocyanate signal at around 2270 $cm^{-1}$ in the IR spectrum), the reaction was stopped by removing the oil bath and cooling the reaction mixture to 25 °C. The product was analysed without further purification.

General procedure 2: Synthesis of polyoxazolidinone 1 from diepoxide 1 and diisocyanate 1 in solvent.

The reactions were performed in a 300 ml stainless steel autoclave as described above, using a motor with a stirring power of 90 W. The reactor was charged with diepoxide 1 (15.0 g, 44.1 mmol) and catalyst and subsequently

flushed with argon. After addition of 40 ml of dry NMP, the reactor was sealed and the mixture was heated to 200 °C under rapid stirring (1000 rpm). After reaching 200 °C, a solution of diisocyanate 1 (7.3 g, 41.97 mmol) in 15 ml dry NMP was added with an HPLC pump (10 ml pump head) over 2.5 h. After an overall reaction time of 19.25 h (starting with addition of diisocyanate 1), the reaction mixture was cooled down to room temperature. The resulting reaction mixture was poured into 150 ml of methanol. The resulting precipitate was collected by filtration and washed with methanol and with diethylether. The obtained powder was dried at $5\times10^{-2}$ mbar for 3 hours.

Example 1:

Synthesis of oxazolidinone 1 from monoepoxide 1 and monoisocyanate 1 in the presence of catalyst 1 (bis-tetraphenylphosphonium carbonate) at 160 °C in the absence of a solvent

The synthesis was performed following the general procedure 1 at 160 °C in the absence of a solvent, using 64.8 mg (0.0877 mmol, 1 mol% with respect to monoepoxide 1) of bis-tetraphenylphosphonium carbonate (catalyst 1). A pale yellow solid was obtained.
Colorimetry: 16 HU
$S_{OXA}$ > 99 %

The formation of side products like isocyanurates, carbodiimides and homo-polymers of the epoxides was not observed.
$TOF_{1/2}$ = 278 $h^{-1}$

Example 2:

Synthesis of oxazolidinone 1 from monoepoxide 1 and monoisocyanate 1 in the presence of catalyst 2 (bis-tetraphenylphosphonium nitrate) at 160 °C in the absence of a solvent

The synthesis was performed following the general procedure 1 without a solvent, using 35.3mg (0.0877 mmol, 1 mol% with respect to monoepoxide 1) of tetraphenylphosphonium nitrate (catalyst 2). A pale yellow solid was obtained.
Colorimetry: 18 HU
$S_{OXA}$ = 96.7 %

The formation of other side products than isocyanurates, such as carbodiimides and homo-polymers of the epoxides was not observed.
$TOF_{1/2}$ = 33 $h^{-1}$

Example 3 (Comparison):

Synthesis of oxazolidinone 1 from monoepoxide 1 and monoisocyanate 1 in the presence of reference catalyst 1 (tetraphenylphosphonium chloride) at 160 °C in the absence of a solvent

The synthesis was performed following the general procedure 1 without a solvent, using 33.1 mg (0.0877 mmol, 1 mol% with respect to monoepoxide 1) of tetraphenylphosphonium chloride (catalyst 3). A yellow solid was obtained.
Colorimetry: 42 HU
$S_{OXA}$ =88.8%
$TOF_{1/2}$ = 12 $h^{-1}$

Example 4 (Comparison):

Synthesis of oxazolidinone 1 from monoepoxide 1 and monoisocyanate 1 in the presence of reference catalyst 2 (tetraphenylphosphonium bromide) at 160 °C in the absence of a solvent

The synthesis was performed following the general procedure 1 without a solvent, using 36.8 mg (0.0877 mmol, 1 mol % with respect to monoepoxide 1) of tetraphenylphosphonium bromide (catalyst 4). A yellow solid was obtained.
Colorimetry: 40 HU
$S_{OXA}$ = 92.6 %
$TOF_{1/2}$ = 80 $h^{-1}$

Table 1: Results of Examples 1 to 4

| Example | Catalyst | Colorimetry [HU] | $S_{OXA}$ [%] | $TOF_{1/2}$ [$h^{-1}$] |
|---|---|---|---|---|
| 1 | Catalyst 1 $(PPh_4)_2CO_3$ | 16 | > 99.0 | 278 |
| 2 | Catalyst 2 $PPh_4NO_3$ | 18 | 96.7 | 33 |
| 3 (comp.) | Catalyst 3 $PPh_4Cl$ | 42 | 88.8 | 12 |
| 4 (comp.) | Catalyst 4 $PPh_4Br$ | 40 | 92.6 | 80 |
| (comp.) = comparison | | | | |

The resulting oxazolidinones prepared from monoepoxides and monoisocyanates in the presence of the inventive catalysts exhibit lower colour intensity (examples 1 and 2) than the ones prepared with the non-inventive catalysts (comparative examples 3 and 4). Furthermore, a higher chemoselectivity $S_{OXA}$ is obtained when the process is performed in the presence of the inventive catalysts (examples 1 and 2).

Example 1 (catalyst 1, according to the invention) compared to examples 3 and 4 (non-inventive catalysts) further demonstrates that a higher turnover frequency $TOF_{1/2}$ is obtained.

Example 5:

Synthesis of oxazolidinone 1 from monoepoxide 1 and monoisocyanate 1 in the presence of catalyst 1 (bis-tetraphenylphosphonium carbonate) at 160 °C in the presence of a solvent

The synthesis was performed following the general procedure 1 in 2 mL sulfolane, using 32.4 mg (0.0439 mmol, 0.5 mol% with respect to monoepoxide 1) of catalyst 1. A pale yellow solution was obtained.
$TOF_{1/2}$ = 206 $h^{-1}$

Example 5 demonstrates that oxazolidinones are obtained at high activity, when the reaction of monoepoxides and monoisocyanates is performed using a catalyst according to the invention in a solvent process.

Example 6:

Synthesis of oxazolidinone 1 from monoepoxide 1 and monoisocyanate 1 in the presence of a different amount of catalyst 1 (bis-tetraphenylphosphonium carbonate) at 160 °C in the absence of a solvent

The synthesis was performed following the general procedure 1 at 160 °C in the absence of a solvent, using 32.4 mg (0.0439 mmol, 0.5 mol% with respect to monoepoxide 1) of catalyst 1. A pale yellow solid was obtained.
Colorimetry: 18 HU
$TOF_{1/2}$ = 190 $h^{-1}$

Table 2: Results of the reaction of monoisocyanate 1 and monoepoxide 1 in the presence of catalyst 1 at different catalyst concentrations.

| Example | Catalyst concentration | Colorimetry [HU] | $TOF_{1/2}$ [$h^{-1}$] |
|---|---|---|---|
| 1 | 1 mol % | 16 | 278 |
| 6 | 0.5 mol % | 18 | 190 |

Example 6 demonstrates that catalyst 1 (catalyst according to the invention) shows good activity (high $TOF_{1/2}$) and affords oxazolidinones with a low colour intensity also at reduced catalyst concentration.

Examples 7 and 8:

Synthesis of oxazolidinone 1 from monoepoxide 1 and monoisocyanate 1 in the presence of catalyst 1 (bis-tetraphenylphosphonium carbonate) at different temperatures

Examples 7 and 8 were preformed following general procedure 1 in the absence of a solvent at different temperatures. The catalyst used in both examples was catalyst 1 (64.8 mg, 0.0877 mmol, 1 mol% with respect to

monoepoxide 1). The results are presented in Table 3.

Table 3: Reaction of monoisocyanate 1 and monoepoxide 1 in the presence of catalyst 1 under solvent-free conditions at different reaction temperatures.

| Example | Temperature [°C] | $TOF_{1/2}$ [$h^{-1}$] |
|---|---|---|
| 7 | 140 | 83 |
| 1 | 160 | 278 |
| 8 | 180 | 327 |

The results of Table 3 are in accordance with the general teaching that the catalyst activity increases with the temperature. These examples demonstrate that the reaction may be also performed at higher temperatures than 160 °C.

Example 9:

Synthesis of polyoxazolidinone 1 from diepoxide 1 and diisocyanate 1 in the presence of catalyst 1 (bis-tetraphenylphosphonium carbonate) at 200 °C in the presence of a solvent

The synthesis was performed following the general procedure 2 using 15.0 g (44.1 mmol) of diepoxide 1, 342 mg (0.46 mmol) of catalyst 1 (bis-tetraphenylphosphonium carbonate), 7.30 g (41.97 mol) of diisocyanate 1 in 60 mL of dry NMP. A pale brown powder (16.3 g) was obtained.

The presence of oxazolidinone moieties was confirmed by the characteristic IR-signal at v=1749 $cm^{-1}$ The IR-spectra did not show the characteristic isocyanurate-band in the region of 1690-1710 $cm^{-1}$, thus the chemoselectivity was > 96 %. The formation of other side products, such as carbodiimides and homo-polymers of the epoxides was not observed.
Colorimetry: 10.7 CVI
$M_n$(GPC): 3370 g/mol
$T_D$: 402°C
$T_g$: 169°C

Example 10 (Comparison):

Synthesis of polyoxazolidinone 1 from diepoxide 1 and diisocyanate 1 in the presence of catalyst 4 (tetraphenylphosphonium bromide) at 200 °C in the presence of a solvent

The synthesis was performed following the general procedure 1 using 15.0 g (44.1 mmol) of diepoxide 1, 194 mg (0.46 mmol) of catalyst 4 (tetraphenylphosphonium bromide) and 7.30 g (41.97mol) of diisocyanate 1. A pale brown powder (14.6 g) was obtained.

The presence of oxazolidinone moieties was confirmed by the characteristic IR-signal at v=1746 $cm^{-1}$ The IR-spectra did not show the characteristic isocyanurate-band in the region of 1690-1710 $cm^{-1}$.
Colorimetry: 21.3 CVI
$M_n$(GPC): 3390 g/mol
$T_D$: 409°C
$T_g$: 155°C

Table 4: Comparison of polyoxyzolidinone products obtained from reaction of diisocyanate 1 and diepoxide 1 by use of catalyst 1 and catalyst 4.

| Example | Catalyst | Colorimetry [CVI] | $T_g$ [°C] |
|---|---|---|---|
| 9 | Catalyst 1 $(PPh_4)_2CO_3$ | 10.7 | 169 |
| 10 (comp.) | Catalyst 4 $PPh_4Br$ | 21.3 | 155 |
| (comp.) = comparison | | | |

The comparison of example 9 (catalyst 1, according to the invention) with comparison example 10 (catalyst 4) demonstrates that under otherwise equal reaction conditions, polyoxazolidinones with lower colour intensity are obtained,

when a catalyst according to the invention is employed.

Example 11:

Synthesis of polyoxazolidinone 2 from diepoxide 1, diisocyanate 1 and monoisocyanate 2 in the presence of catalyst 1 (bis-tetraphenylphosphonium carbonate) at 200 °C in the presence of a solvent

The synthesis was performed following the general procedure 2 using 15.0 g (44.1 mmol) of diepoxide 1, 342 mg (0.46 mmol) of catalyst 1 (bis-tetraphenylphosphonium carbonate), 7.30 g (41.97 mmol) of diisocyanate 1 and 900 mg (4.76 mmol) of monoisocyanate 2 in 60 mL of a mixture of *ortho*-dichlorobenzene and NMP in a volume ratio of 1:5. A pale brown powder was obtained.

The presence of oxazolidinone moieties was confirmed by the characteristic IR-signal at $\nu$=1749 $cm^{-1}$.

Colorimetry: 4.3 CVI
$M_n$(GPC): 4821 g/mol
$T_D$: 404°C
$T_g$: 153°C

Example 11 demonstrates that polyoxazolidinone compounds are obtained, when diepoxides, diisocyanates, and monoisocyanates are reacted in the presence of a catalyst according to the invention.

**Claims**

1. A method for the production of oxazolidinone compounds, comprising the step of reacting an epoxide compound and an isocyanate compound in the presence of a catalyst,
**characterized in that** the catalyst
is free of halide anions, and
comprises a conjugated trigonal-planar anionic moiety having a central atom selected from the elements C or N and three further substituent atoms, selected independently of one another from the elements C, N, O, S and/or P, and wherein the overall charge state of the conjugated trigonal-planar anionic moiety is -1 or -2.

2. A method according to claim 1, wherein the anionic moiety in the catalyst has the general formula (V),

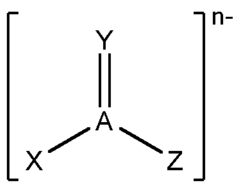

(V)

wherein

n is an integer of 1 or 2,
A is C or $N^+$,
X is $O^-$, OH, $S^-$, SH, OR, SR, NRR', NHR, $N(R)^-$, $N(SO_2R)^-$ or PRR',
wherein R, R' independently of one another are linear or branched, saturated, mono- or polyunsaturated aliphatic, cycloaliphatic or aromatic groups comprising 1 to 20 carbon atoms, optionally substituted with one or more heteroatom containing groups, wherein the heteroatom is selected from N, O, S, P, Si, F or Cl,
wherein R, R' can be connected within a (hetero)aliphatic or (hetero)aromatic mono- or polycyclic ring comprising 1 to 20 carbon atoms and/or heteroatoms selected from the group N, P, O and/or S optionally substituted with one or more heteroatom containing groups, wherein the heteroatom is selected from N, O, S, P, Si, F and/or Cl, or with an alkyl group comprising 1 to 20 carbon atoms,
Y is O, S or N, wherein N can be substituted by a group R", wherein R" independently of R and R' has the same meaning as specified for R, R',
Z is $-O^-$, $S^-$, $N(R''')^-$, $N(SO_2R''')^-$, wherein R''' independently of R, R' and R" has the same meaning as specified for R, R', R", or a carbon atom which is part of an aromatic group comprising 1 to 20 carbon atoms,
wherein in case that Y is N, Y and X can be connected within a (hetero)aliphatic or (hetero)aromatic ring having

5 to 6 carbon atoms and/or heteroatoms selected from the group N, P, O and/or S and optionally comprising an annulated aromatic ring having 6 carbon atoms optionally substituted with one or more heteroatom containing groups, wherein the heteroatom is selected from N, O, S, P, Si, F and/or Cl, or with an alkyl group comprising 1 to 20 carbon atoms.

**3.** A method according to claim 1 or 2, wherein the anionic moiety of the catalyst is selected from the group comprising $CO_3^{2-}$, RO-$CO_2^-$, RS-$CO_2^-$, RHN-$CO_2^-$, RR'N-$CO_2^-$, $CSO_2^{2-}$, RO-$CSO^-$, RHN-$CSO^-$, RR'N-$CSO^-$, RS-$CSO^-$, $COS_2^{2-}$, RO-$CS_2^-$, RHN-$CS_2^-$, RR'NT$CS_2^-$, RR'N-$CSO^-$, $CS_3^{2-}$, RS-$CS_2^-$, RO-C(=NR")$O^-$, RO-C(=NR")$S^-$, RS-C(=NR")$O^-$, RS-C(=NR")$S^-$, RN=C(NHR")$O^-$, R"N=C(NRR')$O^-$, R"N=C(NHR)$S^-$, R"N=C(NRR')$S^-$, RO-CO-N(R''')$^-$, RS-CO-N(R''')$^-$, RR'N-CO-N(R''')$^-$, RO-CS-N(R''')$^-$, RS-CS-N(R''')$^-$, RR'N-CS-N(R''')$^-$, RO-C(=NR'')-N(R''')$^-$, RS-C(=NR")-N(R''')$^-$, RR'N-C(=NR")-N(R''')$^-$, RO-CO-N($SO_2$R''')$^-$, RS-CO-N($SO_2$R''')$^-$, RR'N-CO-N($SO_2$R''')$^-$, RO-CS-N($SO_2$R''')$^-$, RS-CS-N($SO_2$R''')$^-$, RR'N-CS-N($SO_2$R''')$^-$, RO-C(=NR'')-N($SO_2$R''')$^-$, RS-C(=NR")-N($SO_2$R''')$^-$, RR'N-C(=NR")-N($SO_2$R''')$^-$,
wherein R, R', R", R''' independently of one another are linear or branched, saturated, mono- or polyunsaturated aliphatic, cycloaliphatic or aromatic groups comprising 1 to 20 carbon atoms, optionally substituted with one or more heteroatom containing groups, wherein the heteroatoms are selected from N, O, S, P, Si, F and/or Cl,
wherein R and R' and/or R''', and/or R' and R" and/or R''' can be connected within a (hetero)aliphatic or (hetero)aromatic, mono- or polycyclic ring system comprising 1 to 20 carbon atoms and/or heteroatoms selected from the group N, P, O and/or S, optionally substituted with one or more heteroatom containing groups, wherein the heteroatom is selected from N, O, S, P, Si, F and/or Cl, or with an alkyl group comprising 1 to 20 carbon atoms,
4,5-dihydro-oxazol-2-olate, optionally substituted in 4- and/or 5-position with an aliphatic, cycloaliphatic or aromatic group having 1 to 20 carbon atoms, oxazol-2-olate, optionally substituted in 4- and/or 5-position with an aliphatic, cycloaliphatic or aromatic group having 1 to 20 carbon atoms, benzoxazol-2-olate, wherein the benzene ring is optionally substituted with one to four aliphatic, cycloaliphatic or aromatic groups having 1 to 20 carbon atoms, 4,5-dihydro-thiazol-2-olate, optionally substituted in 4- and/or 5-position with an aliphatic, cycloaliphatic or aromatic group having 1 to 20 carbon atoms, thiazol-2-olate, optionally substituted in 4- and/or 5-position with an aliphatic, cycloaliphatic or aromatic group having 1 to 20 carbon atoms, benzothiazol-2-olate, wherein the aromatic ring is optionally substituted with one to four aliphatic, cycloaliphatic or aromatic groups having 1 to 20 carbon atoms, 4,5-dihydro-1,3-diazol-2-olate, optionally substituted in 4- and/or 5-position with an aliphatic, cycloaliphatic or aromatic group having 1 to 20 carbon atoms, 1,3-diazol-2-olate, optionally substituted in 4- and/or 5-position with an aliphatic, cycloaliphatic or aromatic group having 1 to 20 carbon atoms, benzo-1,3-diazol-2-olate, wherein the aromatic ring is optionally substituted with one to four aliphatic, cycloaliphatic or aromatic groups having 1 to 20 carbon atoms, 4,5-dihydro-oxazol-2-thiolate, optionally substituted in 4- and/or 5-position with an aliphatic, cycloaliphatic or aromatic group having 1 to 20 carbon atoms, oxazol-2-thiolate, optionally substituted in 4- and/or 5-position with an aliphatic, cycloaliphatic or aromatic group having 1 to 20 carbon atoms, benzoxazol-2-thiolate, wherein the aromatic moiety is optionally substituted with one to four aliphatic, cycloaliphatic or aromatic groups having 1 to 20 carbon atoms, 4,5-dihydro-thiazol-2-thiolate, optionally substituted in 4- and/or 5-position with an aliphatic, cycloaliphatic or aromatic group having 1 to 20 carbon atoms, thiazol-2-thiolate, optionally substituted in 4- and/or 5-position with an aliphatic, cycloaliphatic or aromatic group having 1 to 20 carbon atoms, benzothiazol-2-thiolate, wherein the aromatic moiety is optionally substituted with one to four aliphatic, cycloaliphatic or aromatic groups having 1 to 20 carbon atoms, 4,5-dihydro-1,3-diazol-2-thiolate, optionally substituted in 4- and/or 5-position with an aliphatic, cycloaliphatic or aromatic group having 1 to 20 carbon atoms, 1,3-diazol-2-thiolate, optionally substituted in 4- and/or 5-position with an aliphatic, cycloaliphatic or aromatic group having 1 to 20 carbon atoms, benzo-1,3-diazol-2-thiolate, wherein the aromatic moiety is optionally substituted with one to four aliphatic, cycloaliphatic or aromatic groups having 1 to 20 carbon atoms, pyrimidin-2-yl amide, optionally *N*-substitued, e.g. with aryl or alkylsulfonyl groups, 2-pyrimidin-2-olate, *N*-alkylsulfonyl or *N*-arylsulfonyl carbamimidoylamide, optionally with further *N*'- and/or *N*"-substituents, or $NO_3^-$.

**4.** A method according to one of the claims 1 to 3, wherein the anionic moiety of the catalyst is selected from the group comprising $CO_3^{2-}$, $CSO_2^{2-}$, $COS_2^{2-}$, RO-$CO_2^-$; wherein R is methyl, ethyl, propyl, isopropyl, butyl or phenyl, RS-$CO_2^-$, wherein R is methyl, ethyl or phenyl, $R_2$N-$CO_2^-$, wherein R is methyl, ethyl or phenyl, $R_2$N-$CSO^-$, wherein R is methyl, ethyl or phenyl, $R_2$N-$CS_2^-$, wherein R is methyl, ethyl or phenyl, RO-$CS_2^-$, wherein R is methyl, ethyl or phenyl, 4,5-dihydro-oxazol-2-thiolate, oxazol-2-thiolate, benzoxazol-2-thiolate, 4,5-dihydro-thiazol-2-thiolate, thiazol-2-thiolate, benzothiazol-2-thiolate, 4,5-dihydro-1,3-diazol-2-thiolate, 1,3-diazol-2-thiolate, benzo-1,3-diazol-2-thiolate or $NO_3^-$.

**5.** A method according to one of the claims 1 to 4, wherein the anionic moiety of the catalyst is selected from the group comprising $CO_3^{2-}$, $NO_3^-$, $CSO_2^{2-}$, $COS_2^{2-}$, $(CH_3)_2$N-$CS_2^-$, $(C_2H_5)_2$N-$CS_2^-$, and benzothiazol-2-thiolate.

**6.** A method according to one of the claims 1 to 5, wherein the anionic moiety of the catalyst is selected from the group comprising $CO_3^{2-}$, $NO_3^-$, $(CH_3)_2N\text{-}CS_2^-$ or $(C_2H_5)_2N\text{-}CS_2^-$.

**7.** A method according to one of the claims 1 to 6, wherein the conjugated trigonal-planar anionic moiety comprised in the catalyst is generated from an appropriate nucleophile and $CO_2$, SCO or $CS_2$.

**8.** A method according to one of the claims 1 to 7, wherein the cationic part of the catalyst is represented by formula (VI)

$$[M(R1)(R2)(R3)(R4)]^+ \qquad (VI)$$

wherein

M is phosphorous or antimony,
(R1), (R2), (R3), (R4) are independently of one another selected from the group comprising linear or branched alkyl groups containing 1 to 22 carbon atoms, optionally substituted with heteroatoms and/or heteroatom containing substituents, cycloaliphatic groups containing 3 to 22 carbon atoms, optionally substituted with heteroatoms and/or heteroatom containing substituents, C1 to C3 alkyl-bridged cycloaliphatic groups containing 3 to 22 carbon atoms, optionally substituted with heteroatoms and/or heteroatom containing substituents and aryl groups containing 6 to 18 carbon atoms, optionally substituted with alkyl groups containing 1 to 10 carbon atoms and/or heteroatoms.

**9.** A method according to one of the claims 1 to 8, wherein the cationic part of the catalyst is represented by formula (VI)

$$[M(R1)(R2)(R3)(R4)]^+ \qquad (VI)$$

wherein

M is phosphorous or antimony,
(R1), (R2), (R3), (R4) are independently of one another selected from the group comprising phenyl, *n*-butyl and cyclohexyl.

**10.** Method according to one or more of claims 1 to 9 wherein the isocyanate compound is added to the epoxide compound in a continuous or step-wise manner with two or more individual addition steps in the step-wise addition, wherein in each individual addition step the amount of isocyanate compound added is $\leq$ 50 weight-% of the total amount of isocyanate compound to be added.

**11.** Method according to one or more of claims 1 to 10 wherein the reaction is conducted at a temperature of $\geq$ 130 °C to $\leq$ 280 °C.

**12.** An oxazolidinone compound, obtainable by a method according to one or more of claims 1 to 11, with a chemoselectivity $S_{OXA}$ to the oxazolidinone product of $\geq$ 93 %.

**13.** An oligomeric or polymeric oxazolidinone compound, obtainable by a method according to one or more of claims 1 to 11 using an isocyanate compound with two or more NCO groups per molecule and an epoxide compound with two or more epoxy groups per molecule, comprising at least one unit derived from the isocyanate compound and at least two units derived from the epoxide compound or comprising at least one unit derived from the epoxide compound and at least two units derived from the isocyanate compound, with a colour intensity of < 21 CVI in reference to the iodine colour scale (DIN 6162).

**14.** The compound according to claim 12 or 13, comprising at least one terminal epoxide and/or isocyanate group or comprising at least one terminal group which is non-reactive towards epoxide and/or isocyanate groups.

**15.** The use of a catalyst in the reaction of isocyanate compounds or polyisocyanate compounds with epoxide compounds or polyepoxide compounds, wherein the catalyst is free of halide anions, and
comprises a conjugated trigonal-planar anionic moiety having a central atom selected from the elements C or N and three further substituent atoms, selected independently of one another from the elements C, N, O, S and/or P, and wherein the overall charge state of the conjugated trigonal-planar anionic moiety is -1 or - 2.

**Patentansprüche**

1. Verfahren zur Herstellung von Oxazolidinonverbindungen, das den Schritt des Umsetzens einer Epoxidverbindung und einer Isocyanatverbindung in Gegenwart eines Katalysators umfasst,
**dadurch gekennzeichnet, dass** der Katalysator frei von Halogenidanionen ist und
eine konjugierte trigonal-planare anionische Gruppierung mit einem Zentralatom, das aus den Elementen C oder N ausgewählt ist, und
drei weitere Substituentenatome, die unabhängig voneinander aus den Elementen C, N, O, S und/oder P ausgewählt sind, umfasst, und
wobei die Gesamtladung der konjugierten trigonalplanaren anionischen Gruppierung -1 oder -2 beträgt.

2. Verfahren nach Anspruch 1, bei dem die anionische Gruppierung in dem Katalysator die allgemeine Formel (V)

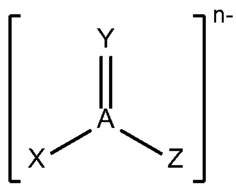

(V)

aufweist, wobei

n für eine ganze Zahl mit einem Wert von 1 oder 2 steht,
A für C oder $N^+$ steht,
X für $O^-$, OH, S-, SH, OR, SR, NRR' , NHR, $N(R)^-$, $N(SO_2R)^-$ oder PRR' steht,
wobei R und R' unabhängig voneinander für lineare oder verzweigte, gesättigte, ein- oder mehrfach ungesättigte aliphatische, cycloaliphatische oder aromatische Gruppen mit 1 bis 20 Kohlenstoffatomen, die gegebenenfalls durch eine oder mehrere heteroatomhaltige Gruppen, wobei das Heteroatom aus N, O, S, P, Si, F oder Cl ausgewählt ist, substituiert sind, stehen,
wobei R und R' in einem (hetero)aliphatischen oder (hetero)aromatischen mono- oder polycyclischen Ring mit 1 bis 20 Kohlenstoffatomen und/oder Heteroatomen, die aus der Gruppe N, P, O und/oder S ausgewählt sind, der gegebenenfalls durch eine oder mehrere heteroatomhaltige Gruppen, wobei das Heteroatom aus N, O, S, P, Si, F und/oder Cl ausgewählt ist, oder durch eine Alkylgruppe mit 1 bis 20 Kohlenstoffatomen substituiert ist, verbunden sein können,
Y für O, S oder N steht, wobei N durch eine Gruppe R'' substituiert sein kann, wobei R" unabhängig von R und R' die gleiche Bedeutung wie für R und R' angegeben aufweist,
Z für $-O^-$, S-, $N(R''')^-$, $N(SO_2R''')^-$, wobei R''' unabhängig von R, R' und R" die gleiche Bedeutung wie für R, R', R" angegeben aufweist, oder ein Kohlenstoffatom, das Teil einer aromatischen Gruppe mit 1 bis 20 Kohlenstoffatomen ist, steht, wobei in dem Fall, dass Y für N steht, Y und X in einem (hetero)aliphatischen oder (hetero)aromatischen Ring mit 5 bis 6 Kohlenstoffatomen und/oder Heteroatomen, die aus der Gruppe N, P, O und/oder S ausgewählt sind, der gegebenenfalls einen anellierten aromatischen Ring mit 6 Kohlenstoffatomen umfasst, der gegebenenfalls durch eine oder mehrere heteroatomhaltige Gruppen, wobei das Heteroatom aus N, O, S, P, Si, F und/oder Cl ausgewählt ist, oder durch eine Alkylgruppe mit 1 bis 20 Kohlenstoffatomen substituiert ist, verbunden sein können.

3. Verfahren nach Anspruch 1 oder 2, bei dem die anionische Gruppierung des Katalysators aus der Gruppe umfassend $CO_3^{2-}$, $RO\text{-}CO_2^-$, $RS\text{-}CO_2^-$, $RHN\text{-}CO_2^-$, $RR'N\text{-}CO_2^-$, $CSO_2^{2-}$, RO-CSO-, RHN-CSO-, $RR'N\text{-}CSO^-$, RS-CSO-, $COS_2^{2-}$, $RO\text{-}CS_2^-$, $RHN\text{-}CS_2^-$, $RR'NCS_2^-$, $RR'N\text{-}CSO^-$, $CS_3^{2-}$, $RS\text{-}CS_2^-$, RO-C(=NR")$O^-$, RO-C(=NR")$S^-$, RS-C(=NR")$O^-$, RS-C(=NR")$S^-$, RN=C(NHR")$O^-$, R"N=C(NRR')$O^-$, R"N=C(NHR)$S^-$, R"N=C(NRR')$S^-$, RO-CO-$N(R''')^-$, RS-CO-$N(R''')^-$, RR'N-CO-$N(R''')^-$, RO-CS-$N(R''')^-$, RS-CS-$N(R''')^-$, RR'N-CS-$N(R''')^-$, RO-C(=NR")-$N(R''')^-$, RS-C(=NR")-N(R''')-, RR'N-C(=NR")-$N(R''')^-$, RO-CO-$N(SO_2R''')^-$, RS-CO-$N(SO_2R''')^-$, RR'N-CO-$N(SO_2R''')^-$, RO-CS-$N(SO_2R''')^-$, RS-CS-$N(SO_2R''')^-$, RR'N-CS-$N(SO_2R''')^-$, RO-C(=NR'')-$N(SO_2R''')^-$, RS-C(=NR")-$N(SO_2R''')^-$, RR'N-C(=NR")-$N(SO_2R''')^-$, wobei R, R', R" und R''' unabhängig voneinander für lineare oder verzweigte, gesättigte, ein- oder mehrfach ungesättigte aliphatische, cycloaliphatische oder aromatische Gruppen mit 1 bis 20 Kohlenstoffatomen, die gegebenenfalls durch eine oder mehrere heteroatomhaltige Gruppen, wobei das Heteroatom aus N, O, S, P, Si, F und/oder Cl ausgewählt ist, substituiert sind, stehen,

wobei R und R' und/oder R''' und/oder R' und R" und/oder R''' in einem (hetero)aliphatischen oder (hetero)aromatischen mono- oder polycyclischen Ringsystem mit 1 bis 20 Kohlenstoffatomen und/oder Heteroatomen, die aus der Gruppe N, P, O und/oder S ausgewählt sind, der gegebenenfalls durch eine oder mehrere heteroatomhaltige Gruppen, wobei das Heteroatom aus N, O, S, P, Si, F und/oder Cl ausgewählt ist, oder durch eine Alkylgruppe mit 1 bis 20 Kohlenstoffatomen substituiert ist, verbunden sein können,
4,5-Dihydrooxazol-2-olat, das gegebenenfalls in 4- und/oder 5-Position mit einer aliphatischen, cycloaliphatischen oder aromatischen Gruppe mit 1 bis 20 Kohlenstoffatomen substituiert ist, Oxazol-2-olat, das gegebenenfalls in 4- und/oder 5-Position mit einer aliphatischen, cycloaliphatischen oder aromatischen Gruppe mit 1 bis 20 Kohlenstoffatomen substituiert ist, Benzoxazol-2-olat, wobei der Benzolring gegebenenfalls durch eine bis vier aliphatische, cycloaliphatischen oder aromatische Gruppen mit 1 bis 20 Kohlenstoffatomen substituiert ist, 4,5-Dihydrothiazol-2-olat, das gegebenenfalls in 4- und/oder 5-Position mit einer aliphatischen, cycloaliphatischen oder aromatischen Gruppe mit 1 bis 20 Kohlenstoffatomen substituiert ist, Thiazol-2-olat, das gegebenenfalls in 4- und/oder 5-Position mit einer aliphatischen, cycloaliphatischen oder aromatischen Gruppe mit 1 bis 20 Kohlenstoffatomen substituiert ist, Benzothiazol-2-olat, wobei der aromatische Ring gegebenenfalls durch eine bis vier aliphatische, cycloaliphatischen oder aromatische Gruppen mit 1 bis 20 Kohlenstoffatomen substituiert ist, 4,5-Dihydro-1,3-diazol-2-olat, das gegebenenfalls in 4- und/oder 5-Position mit einer aliphatischen, cycloaliphatischen oder aromatischen Gruppe mit 1 bis 20 Kohlenstoffatomen substituiert ist, 1,3-Diazol-2-olat, das gegebenenfalls in 4- und/oder 5-Position mit einer aliphatischen, cycloaliphatischen oder aromatischen Gruppe mit 1 bis 20 Kohlenstoffatomen substituiert ist, Benzo-1,3-diazol-2-olat, wobei der aromatische Ring gegebenenfalls durch eine bis vier aliphatische, cycloaliphatische oder aromatische Gruppen mit 1 bis 20 Kohlenstoffatomen substituiert ist, 4,5-Dihydrooxazol-2-thiolat, das gegebenenfalls in 4- und/oder 5-Position mit einer aliphatischen, cycloaliphatischen oder aromatischen Gruppe mit 1 bis 20 Kohlenstoffatomen substituiert ist, Oxazol-2-thiolat, das gegebenenfalls in 4- und/oder 5-Position mit einer aliphatischen, cycloaliphatischen oder aromatischen Gruppe mit 1 bis 20 Kohlenstoffatomen substituiert ist, Benzoxazol-2-thiolate, wobei die aromatische Gruppierung gegebenenfalls durch eine bis vier aliphatische, cycloaliphatische oder aromatische Gruppen mit 1 bis 20 Kohlenstoffatomen substituiert ist, 4,5-Dihydrothiazol-2-thiolat, das gegebenenfalls in 4- und/oder 5-Position mit einer aliphatischen, cycloaliphatischen oder aromatischen Gruppe mit 1 bis 20 Kohlenstoffatomen substituiert ist, Thiazol-2-thiolat, das gegebenenfalls in 4- und/oder 5-Position mit einer aliphatischen, cycloaliphatischen oder aromatischen Gruppe mit 1 bis 20 Kohlenstoffatomen substituiert ist, Benzothiazol-2-thiolat, wobei die aromatische Gruppierung gegebenenfalls durch eine bis vier aliphatische, cycloaliphatische oder aromatische Gruppen mit 1 bis 20 Kohlenstoffatomen substituiert ist, 4,5-Dihydro-1,3-diazol-2-thiolat, das gegebenenfalls in 4- und/oder 5-Position mit einer aliphatischen, cycloaliphatischen oder aromatischen Gruppe mit 1 bis 20 Kohlenstoffatomen substituiert ist, 1,3-Diazol-2-thiolat, das gegebenenfalls in 4- und/oder 5-Position mit einer aliphatischen, cycloaliphatischen oder aromatischen Gruppe mit 1 bis 20 Kohlenstoffatomen substituiert ist, Benzo-1,3-diazol-2-thiolat, wobei die aromatische Gruppierung gegebenenfalls durch eine bis vier aliphatische, cycloaliphatische oder aromatische Gruppen mit 1 bis 20 Kohlenstoffatomen substituiert ist, Pyrimidin-2-ylamid, das gegebenenfalls *N*-substituiert ist, z. B. durch Aryl- oder Alkylsulfonylgruppen, 2-Pyrimidin-2-olat, *N*-Alkylsulfonyl- oder *N*-Arylsulfonylcarbamimidoylamid, das gegebenenfalls durch weitere *N'*- und/oder *N*"-Substituenten substituiert ist, oder $NO_3$- ausgewählt ist.

**4.** Verfahren nach einem der Ansprüche 1 bis 3, wobei die anionische Gruppierung des Katalysators aus der Gruppe umfassend $CO_3^{2-}$, $CSO_2^{2-}$, $COS_2^{2-}$, RO-$CO_2^-$, wobei R für Methyl, Ethyl, Propyl, Isopropyl, Butyl oder Phenyl steht, RS-$CO_2^-$, wobei R für Methyl, Ethyl oder Phenyl steht, $R_2$N-$CO_2^-$, wobei R für Methyl, Ethyl oder Phenyl steht, $R_2$N-$CSO^-$, wobei R für Methyl, Ethyl oder Phenyl steht, $R_2$N-$CS_2^-$, wobei R für Methyl, Ethyl oder Phenyl steht, RO-$CS_2^-$, wobei R für Methyl, Ethyl oder Phenyl steht, 4,5-Dihydrooxazol-2-thiolat, Oxazol-2-thiolat, Benzoxazol-2-thiolat, 4,5-Dihydrothiazol-2-thiolat, Thiazol-2-thiolat, Benzothiazol-2-thiolat, 4,5-Dihydro-1,3-diazol-2-thiolat, 1,3-Diazol-2-thiolat, Benzo-1,3-diazol-2-thiolat oder $NO_3$- ausgewählt ist.

**5.** Verfahren nach einem der Ansprüche 1 bis 4, wobei die anionische Gruppierung des Katalysators aus der Gruppe umfassend $CO_3^{2-}$, $NO_3^-$, $CSO_2^{2-}$, $COS_2^{2-}$, $(CH_3)_2N$-$CS_2^-$, $(C_2H_5)_2N$-$CS_2^-$ und Benzothiazol-2-thiolat ausgewählt ist.

**6.** Verfahren nach einem der Ansprüche 1 bis 5, wobei die anionische Gruppierung des Katalysators aus der Gruppe umfassend $CO_3^{2-}$, $NO_3^-$, $(CH_3)_2N$-$CS_2$- oder $(C_2H_5)_2N$-$CS_2^-$ ausgewählt ist.

**7.** Verfahren nach einem der Ansprüche 1 bis 6, bei dem die in dem Katalysator enthaltene konjugierte trigonal-planare anionische Gruppierung aus einem entsprechenden Nucleophil und $CO_2$, SCO oder $CS_2$ erzeugt wird.

**8.** Verfahren nach einem der Ansprüche 1 bis 7, bei dem der kationische Teil des Katalysators durch Formel (VI)

$[M(R1)(R2)(R3)(R4)]^+$ (VI)

wiedergegeben wird, wobei

M für Phosphor oder Antimon steht,
(R1), (R2), (R3), (R4) unabhängig voneinander aus der Gruppe umfassend lineare oder verzweigte Alkylgruppen mit 1 bis 22 Kohlenstoffatomen, die gegebenenfalls durch Heteroatome und/oder heteroatomhaltige Substituenten substituiert sind, cycloaliphatische Gruppen mit 3 bis 22 Kohlenstoffatomen, die gegebenenfalls durch Heteroatome und/oder heteroatomhaltige Substituenten substituiert sind, C1- bis C3-alkylverbrückte cycloaliphatische Gruppen mit 3 bis 22 Kohlenstoffatomen, die gegebenenfalls durch Heteroatome und/oder heteroatomhaltige Substituenten substituiert sind, und Arylgruppen mit 6 bis 18 Kohlenstoffatomen, die gegebenenfalls durch Alkylgruppen mit 1 bis 10 Kohlenstoffatomen und/oder Heteroatome substituiert sind, ausgewählt sind.

**9.** Verfahren nach einem der Ansprüche 1 bis 8, bei dem der kationische Teil des Katalysators durch Formel (VI)

$[M(R1)(R2)(R3)(R4)]^+$ (VI)

wiedergegeben wird, wobei

M für Phosphor oder Antimon steht,
(R1), (R2), (R3), (R4) unabhängig voneinander aus der Gruppe umfassend Phenyl, n-Butyl und Cyclohexyl ausgewählt sind.

**10.** Verfahren nach einem oder mehreren der Ansprüche 1 bis 9, bei dem die man die Isocyanatverbindung kontinuierlich oder schrittweise zu der Epoxidverbindung gibt, wobei die schrittweise Zugabe zwei oder mehr einzelne Zugabeschritte umfasst,
wobei bei jedem einzelnen Zugabeschritt die zugegebene Menge an Isocyanatverbindung ≤ 50 Gew.-% der zuzugebenden Gesamtmenge an Isocyanatverbindung ist.

**11.** Verfahren nach einem oder mehreren der Ansprüche 1 bis 10, bei dem man die Umsetzung bei einer Temperatur von ≥ 130 °C bis ≤ 280 °C durchführt.

**12.** Oxazolidinonverbindung, die durch ein Verfahren nach einem oder mehreren der Ansprüche 1 bis 11 erhältlich ist, mit einer Chemoselektivität $S_{OXA}$ für das Oxazolidinonprodukt > 93 %.

**13.** Oligomere oder polymere Oxazolidinonverbindung, die durch ein Verfahren nach einem der Ansprüche 1 bis 11 unter Verwendung einer Isocyanatverbindung mit zwei oder mehr NCO-Gruppen pro Molekül und einer Epoxidverbindung mit zwei oder mehr Epoxidgruppen pro Molekül erhältlich ist, und mindestens eine Einheit, die sich von der Isocyanatverbindung ableitet, und mindestens zwei Einheiten, die sich von der Epoxidverbindung ableiten, umfasst oder mindestens eine Einheit, die sich von der Epoxidverbindung ableitet, und mindestens zwei Einheiten, die sich von der Isocyanatverbindung ableiten, umfasst, mit einer Farbintensität < 21 CVI in Bezug auf die Iodfarbskala (DIN 6162).

**14.** Verbindung nach Anspruch 12 oder 13, die mindestens eine endständige Epoxid- und/oder Isocyanatgruppe umfasst oder mindestens eine endständige Gruppe, die gegenüber Epoxid- und/oder Isocyanatgruppen unreaktiv ist, umfasst.

**15.** Verwendung eines Katalysators bei der Umsetzung von Isocyanatverbindungen oder Polyisocyanatverbindungen mit Epoxidverbindungen oder Polyepoxidverbindungen, wobei der Katalysator frei von Halogenidanionen ist und eine konjugierte trigonal-planare anionische Gruppierung mit einem Zentralatom, das aus den Elementen C oder N ausgewählt ist, und
drei weitere Substituentenatome, die unabhängig voneinander aus den Elementen C, N, O, S und/oder P ausgewählt sind, umfasst, und
wobei die Gesamtladung der konjugierten trigonalplanaren anionischen Gruppierung -1 oder -2 beträgt.

## Revendications

1. Méthode de production de composés d'oxazolidinone, comprenant l'étape consistant à réagir un composé d'époxyde et un composé d'isocyanate en présence d'un catalyseur, **caractérisée en ce que** le catalyseur est dépourvu d'anions halogénure, et
comprend un motif anionique trigonal planaire conjugué ayant un atome central choisi parmi les éléments C ou N, et trois autres atomes substituants, choisis indépendamment les uns des autres parmi les éléments C, N, O, S et/ou P, et où l'état de charge global du motif anionique trigonal planaire conjugué est de -1 ou -2.

2. Méthode selon la revendication 1, dans laquelle le motif anionique dans le catalyseur répond à la formule générale (V),

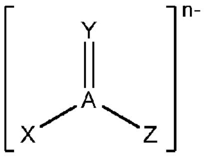

(V)

où

n est un nombre entier valant 1 ou 2,
A est C ou $N^+$,
X est $O^-$, OH, $S^-$, SH, OR, SR, NRR', NHR, $N(R)^-$, $N(SO_2R)^-$ ou PRR',
où R et R', indépendamment l'un de l'autre, sont des groupements linéaires ou ramifiés, saturés, mono- ou polyinsaturés aliphatiques, cycloaliphatiques ou aromatiques comprenant de 1 à 20 atomes de carbone, éventuellement substitués par un ou plusieurs groupements contenant des hétéroatomes, où l'hétéroatome est choisi parmi N, O, S, P, Si, F ou Cl, où R et R' peuvent être reliés au sein d'un cycle (hétéro)aliphatique ou (hétéro)aromatique mono- ou polycyclique comprenant de 1 à 20 atomes de carbone et/ou hétéroatomes choisis dans le groupe constitué par N, P, O et/ou S éventuellement substitué par un ou plusieurs groupements contenant des hétéroatomes, où l'hétéroatome est choisi parmi N, O, S, P, Si, F et/ou Cl, ou par un groupement alkyle comprenant de 1 à 20 atomes de carbone,
Y est O, S ou N, où N peut être substitué par un groupement R", où R", indépendamment de R et R', possède la même signification que celle spécifiée pour R et R',
Z est $-O^-$, $S^-$, $N(R''')^-$, $N(SO_2R''')^-$, où R''', indépendamment de R, R' et R", possède la même signification que celle spécifiée pour R, R', R", ou un atome de carbone qui fait partie d'un groupement aromatique comprenant de 1 à 20 atomes de carbone,
où lorsque Y est N, Y et X peuvent être reliés au sein d'un cycle (hétéro)aliphatique ou (hétéro)aromatique ayant de 5 à 6 atomes de carbone et/ou hétéroatomes choisis dans le groupe constitué par N, P, O et/ou S, et comprenant éventuellement un cycle aromatique annelé ayant 6 atomes de carbone éventuellement substitué par un ou plusieurs groupements contenant des hétéroatomes, où l'hétéroatome est choisi parmi N, O, S, P, Si, F et/ou Cl, ou par un groupement alkyle comprenant de 1 à 20 atomes de carbone.

3. Méthode selon la revendication 1 ou 2, dans laquelle le motif anionique du catalyseur est choisi dans le groupe constitué par
$CO_3^{2-}$, RO-$CO_2^-$, RS-$CO_2^-$, RHN-$CO_2^-$, RR'N-$CO_2^-$, $CSO_2^{2-}$, RO-CSO-, RHN-CSO-, RR'N-$CSO^-$, RS-CSO-, $COS_2^{2-}$, RO-$CS_2^-$, RHN-$CS_2^-$, RR'N$CS_2^-$, RR'N-$CSO^-$, $CS_3^{2-}$, RS-$CS_2^-$, RO-C (=NR'') $O^-$, RO-C(=NR")$S^-$, RS-C(=NR")$O^-$, RS-C(=NR'')$S^-$, RN=C(NHR")$O^-$, R"N=C(NRR')$O^-$, R''N=C(NHR)$S^-$, R"N=C(NRR')S-, RO-CO-$N(R''')^-$, RS-CO-$N(R''')^-$, RR'N-$CON(R''')^-$, RO-CS-$N(R''')^-$, RS-CS-$N(R''')^-$, RR'N-CS-$N(R''')^-$, RO-C(=NR")-$N(R''')^-$, RS-C(=NR")-$N(R''')^-$, RR'N-C(=NR")-$N(R''')^-$, RO-CO-$N(SO_2R''')^-$, RS-$CON(SO_2R''')^-$, RR' N-CO-N $(SO_2R''')^-$, RO-CS-N $(SO_2R''')^-$, RS-CS-$N(SO_2R''')^-$, RR'N-CS-$N(SO_2R''')^-$, RO-C(=NR")-$N(SO_2R''')^-$, RS-C(=NR")-$N(SO_2R''')^-$, RR'N-C(=NR")-$N(SO_2R''')^-$,
où R, R', R" et R''', indépendamment les uns des autres, sont des groupements linéaires ou ramifiés, saturés, mono- ou polyinsaturés aliphatiques, cycloaliphatiques ou aromatiques comprenant de 1 à 20 atomes de carbone, éventuellement substitués par un ou plusieurs groupements contenant des hétéroatomes, où les hétéroatomes sont choisis parmi N, O, S, P, Si, F et/ou Cl,
où R et R' et/ou R''', et/ou R' et R'' et/ou R''' peuvent être reliés au sein d'un noyau (hétéro)aliphatique ou (hétéro)aromatique, mono- ou polycyclique comprenant de 1 à 20 atomes de carbone et/ou hétéroatomes choisis dans

le groupe constitué par N, P, O et/ou S, éventuellement substitué par un ou plusieurs groupements contenant des hétéroatomes, où l'hétéroatome est choisi parmi N, O, S, P, Si, F et/ou Cl, ou par un groupement alkyle comprenant de 1 à 20 atomes de carbone,
4,5-dihydro-oxazol-2-olate, éventuellement substitué en position 4 et/ou 5 par un groupement aliphatique, cycloaliphatique ou aromatique ayant de 1 à 20 atomes de carbone, oxazol-2-olate, éventuellement substitué en position 4 et/ou 5 par un groupement aliphatique, cycloaliphatique ou aromatique ayant de 1 à 20 atomes de carbone, benzoxazol-2-olate, où le cycle benzène est éventuellement substitué par d'un à quatre groupements aliphatiques, cycloaliphatiques ou aromatiques ayant de 1 à 20 atomes de carbone, 4,5-dihydro-thiazol-2-olate, éventuellement substitué en position 4 et/ou 5 par un groupement aliphatique, cycloaliphatique ou aromatique ayant de 1 à 20 atomes de carbone, thiazol-2-olate, éventuellement substitué en position 4 et/ou 5 par un groupement aliphatique, cycloaliphatique ou aromatique ayant de 1 à 20 atomes de carbone, benzothiazol-2-olate, où le cycle aromatique est éventuellement substitué par d'un à quatre groupements aliphatiques, cycloaliphatiques ou aromatiques ayant de 1 à 20 atomes de carbone, 4,5-dihydro-1,3-diazol-2-olate, éventuellement substitué en position 4 et/ou 5 par un groupement aliphatique, cycloaliphatique ou aromatique ayant de 1 à 20 atomes de carbone, 1,3-diazol-2-olate, éventuellement substitué en position 4 et/ou 5 par un groupement aliphatique, cycloaliphatique ou aromatique ayant de 1 à 20 atomes de carbone, benzo-1,3-diazol-2-olate, où le cycle aromatique est éventuellement substitué par d'un à quatre groupements aliphatiques, cycloaliphatiques ou aromatiques ayant de 1 à 20 atomes de carbone, 4,5-dihydro-oxazol-2-thiolate, éventuellement substitué en position 4 et/ou 5 par un groupement aliphatique, cycloaliphatique ou aromatique ayant de 1 à 20 atomes de carbone, oxazol-2-thiolate, éventuellement substitué en position 4 et/ou 5 par un groupement aliphatique, cycloaliphatique ou aromatique ayant de 1 à 20 atomes de carbone, benzoxazol-2-thiolate, où le motif aromatique est éventuellement substitué par d'un à quatre groupements aliphatiques, cycloaliphatiques ou aromatiques ayant de 1 à 20 atomes de carbone, 4,5-dihydro-thiazol-2-thiolate, éventuellement substitué en position 4 et/ou 5 par un groupement aliphatique, cycloaliphatique ou aromatique ayant de 1 à 20 atomes de carbone, thiazol-2-thiolate, éventuellement substitué en position 4 et/ou 5 par un groupement aliphatique, cycloaliphatique ou aromatique ayant de 1 à 20 atomes de carbone, benzothiazol-2-thiolate, où le motif aromatique est éventuellement substitué par d'un à quatre groupements aliphatiques, cycloaliphatiques ou aromatiques ayant de 1 à 20 atomes de carbone, 4,5-dihydro-1,3-diazol-2-thiolate, éventuellement substitué en position 4 et/ou 5 par un groupement aliphatique, cycloaliphatique ou aromatique ayant de 1 à 20 atomes de carbone, 1,3-diazol-2-thiolate, éventuellement substitué en position 4 et/ou 5 par un groupement aliphatique, cycloaliphatique ou aromatique ayant de 1 à 20 atomes de carbone, benzo-1,3-diazol-2-thiolate, où le motif aromatique est éventuellement substitué par d'un à quatre groupements aliphatiques, cycloaliphatiques ou aromatiques ayant de 1 à 20 atomes de carbone, pyrimidin-2-ylamide, éventuellement N-substitué, par exemple par des groupements aryle ou alkylsulfonyle, 2-pyrimidin-2-olate, N-alkylsulfonyle ou N-arylsulfonyl carbamimidoylamide, éventuellement par d'autres substituants N' et/ou N" , ou $NO_3^-$.

**4.** Méthode selon l'une des revendications 1 à 3, dans laquelle le motif anionique du catalyseur est choisi dans le groupe constitué par $CO_3^{2-}$, $CSO_2^{2-}$, $COS_2^{2-}$, RO-$CO_2^-$ où R est méthyle, éthyle, propyle, isopropyle, butyle ou phényle, RS-$CO_2^-$ où R est méthyle, éthyle ou phényle, $R_2$N-$CO_2^-$ où R est méthyle, éthyle ou phényle, $R_2$N-$CSO^-$ où R est méthyle, éthyle ou phényle, $R_2$N-$CS_2^-$ où R est méthyle, éthyle ou phényle, RO-$CS_2^-$, où R est méthyle, éthyle ou phényle, 4,5-dihydro-oxazol-2-thiolate, oxazol-2-thiolate, benzoxazol-2-thiolate, 4,5-dihydro-thiazol-2-thiolate, thiazol-2-thiolate, benzothiazol-2-thiolate, 4,5-dihydro-1,3-diazol-2-thiolate, 1,3-diazol-2-thiolate, benzo-1,3-diazol-2-thiolate ou $NO_3^-$.

**5.** Méthode selon l'une des revendications 1 à 4, dans laquelle le motif anionique du catalyseur est choisi dans le groupe constitué par $CO_3^{2-}$, $NO_3^-$, $CSO_2^{2-}$, $COS_2^{2-}$, $(CH_3)_2N$-$CS_2^-$, $(C_2H_5)_2N$-$CS_2^-$ et benzothiazol-2-thiolate.

**6.** Méthode selon l'une des revendications 1 à 5, dans laquelle le motif anionique du catalyseur est choisi dans le groupe constitué par $CO_3^{2-}$, $NO_3^-$, $(CH_3)_2N$-$CS_2^-$ et $(C_2H_5)_2N$-$CS_2^-$.

**7.** Méthode selon l'une des revendications 1 à 6, dans laquelle le motif anionique trigonal planaire conjugué compris dans le catalyseur est généré à partir d'un nucléophile approprié et $CO_2$, SCO ou $CS_2$.

**8.** Méthode selon l'une des revendications 1 à 7, dans laquelle la partie cationique du catalyseur est représentée par la formule (VI)

$$[M(R1)\ (R2)\ (R3)\ (R4)]^+ \qquad (VI)$$

où

M est phosphore ou antimoine,
(R1), (R2), (R3) et (R4) sont, indépendamment les uns des autres, choisis dans le groupe constitué par des groupements alkyle linéaires ou ramifiés contenant de 1 à 22 atomes de carbone, éventuellement substitués par des hétéroatomes et/ou des substituants contenant des hétéroatomes, des groupements cycloaliphatiques contenant de 3 à 22 atomes de carbone, éventuellement substitués par des hétéroatomes et/ou des substituants contenant des hétéroatomes, des groupements cycloaliphatiques pontés par alkyle en C1 à C3 contenant de 3 à 22 atomes de carbone, éventuellement substitués par des hétéroatomes et/ou des substituants contenant des hétéroatomes et des groupements aryle contenant de 6 à 18 atomes de carbone, éventuellement substitués par des groupements alkyle contenant de 1 à 10 atomes de carbone et/ou hétéroatomes.

**9.** Méthode selon l'une des revendications 1 à 8, dans laquelle la partie cationique du catalyseur est représentée par la formule (VI)

$$[M(R1) (R2) (R3) (R4)]^+ \qquad (VI)$$

où

M est phosphore ou antimoine,
(R1), (R2), (R3) et (R4) sont, indépendamment les uns des autres, choisis dans le groupe constitué par phényle, n-butyle et cyclohexyle.

**10.** Méthode selon l'une ou plusieurs parmi les revendications 1 à 9, dans laquelle le composé d'isocyanate est ajouté au composé d'époxyde de manière continue ou par étapes, avec deux, ou plus, étapes d'addition individuelles dans l'addition par étapes,
où dans chaque étape d'addition individuelle, la quantité de composé d'isocyanate ajoutée est ≤50% en poids de la quantité totale du composé d'isocyanate devant être ajoutée.

**11.** Méthode selon l'une ou plusieurs parmi les revendications 1 à 10, dans laquelle la réaction est effectuée à une température allant de ≥130°C, à ≥280°C.

**12.** Composé d'oxazolidinone, pouvant être obtenu par une méthode selon l'une ou plusieurs parmi les revendications 1 à 11, ayant une chimiosélectivité $S_{OXA}$ par rapport au produit d'oxazolidinone >93%.

**13.** Composé d'oxazolidinone oligomère ou polymère, pouvant être obtenu par une méthode selon l'une ou plusieurs parmi les revendications 1 à 11, en utilisant un composé d'isocyanate ayant deux, ou plus, groupements NCO par molécule et un composé d'époxyde ayant deux, ou plus, groupements époxy par molécule, comprenant au moins une unité dérivée du composé d'isocyanate et au moins deux unités dérivées du composé d'époxyde, ou comprenant au moins une unité dérivée du composé d'époxyde et au moins deux unités dérivées du composé d'isocyanate, ayant une intensité de couleur <21 CVI en faisant référence à l'échelle de couleur d'iode (DIN 6162).

**14.** Composé selon la revendication 12 ou 13, comprenant au moins un groupement époxyde et/ou isocyanate terminal, ou comprenant au moins un groupement terminal qui est non réactif vis-à-vis des groupements époxyde et/ou isocyanate.

**15.** Utilisation d'un catalyseur dans la réaction de composés d'isocyanate ou de composés de polyisocyanate avec des composés d'époxyde ou des composés de polyépoxyde, où le catalyseur est dépourvu d'anions halogénure, et comprend un motif anionique trigonal planaire conjugué ayant un atome central choisi parmi les éléments C ou N, et trois autres atomes substituants, choisis indépendamment les uns des autres parmi les éléments C, N, O, S et/ou P, et où l'état de charge global du motif anionique trigonal planaire conjugué est de -1 ou -2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5480958 A
- US 2014121299 A1
- US 2010227090 A1
- EP 12192611 A
- US 3687897 A


### Non-patent literature cited in the description


- **M. E. DYEN ; D. SWERN.** *Chem. Rev.,* 1967, vol. 67, 197
- **X. ZHANG ; W. CHEN.** *Chem. Lett.,* 2010, vol. 39, 527
- **M.T. BARROS ; A.M.F. PHILLIPS.** *Tetrahedron: Asymmetry,* 2010, vol. 21, 2746
- **H.-Y. WU ; J.-C. DING ; Y.-K. LIU.** *J. Indian Chem. Soc.,* 2003, vol. 80, 36
- **C. QIAN ; D. ZHU.** *Synlett,* 1994, 129
- **HOLLEMAN, A. F. ; WIBERG, E.** Inorganic Chemistry. Academic Press, 2001
- **E. KRIESTEN ; D. MAYER ; F. ALSMEYER ; C. B. MINNICH ; L. GREINER ; W. MARQUARDT.** *Chemomet. Intell. Lab. Sys.,* 2008, vol. 93 (2), 108